# EUROPEAN PATENT SPECIFICATION

(11) **EP 4 327 239 B1**
(45) Date of publication and mention of the grant of the patent: **19.08.2026**
(21) Application number: 21942846.3
(22) Date of filing: 30.12.2021
(51) Int. Cl.: G06T 11/00, G01T 1/164, G01T 1/29, A61B 6/00

(54) **SYSTEMS AND METHODS FOR DATA ACQUISITION AND TRANSMISSION IN PET**
SYSTEME UND VERFAHREN ZUR DATENERFASSUNG UND -ÜBERTRAGUNG IN PET
SYSTÈMES ET PROCÉDÉS D'ACQUISITION ET DE TRANSMISSION DE DONNÉES EN PET

(30) Priority: 25.05.2021 CN 202110571764; 25.06.2021 CN 202110713880
(43) Date of publication of application: 28.02.2024
(73) Proprietor: Shanghai United Imaging Healthcare Co., Ltd., Shanghai 201807 (CN)
(72) Inventor: LI, Jun, Shanghai 201807 (CN); ZHENG, Wei, Shanghai 201807 (CN); SUN, Youjun, Shanghai 201807 (CN)
(74) Representative: Wang, Bo
(86) International application number: PCT/CN2021/143420
(87) International publication number: WO 2022/247283

(56) References cited:
- CN-A- 109 658 473
- CN-A- 110 584 698
- CN-A- 111 402 355
- CN-A- 113 393 547
- CN-A- 113 538 614
- US-A1- 2008 317 194
- US-A1- 2010 202 664
- US-A1- 2012 061 576
- US-A1- 2012 089 015
- US-A1- 2014 257 096
- US-A1- 2016 306 054
- US-A1- 2020 151 918

## Description

### CROSS-REFERENCE TO RELATED APPLICATIONS

This application claims priority to Chinese Patent Application No. 202110713880.7, filed on June 25, 2021, and Chinese Patent Application No. 202110571764.6, filed on May 25, 2021.

### TECHNICAL FIELD

The present invention generally relates to data acquisition, and more specifically relates to systems and methods for data acquisition and transmission in PET.

### BACKGROUND

A positron emission computed tomography (PET) technology has been widely used in clinical examination and disease diagnosis. In some occasions, when images of a specific region (e.g., the head, a liver, etc.) of an object (e.g., a patient) are needed for medical diagnosis and/or treatment, image data of the object obtained by a PET device may include data of other regions except for the specific region, which results in a large amount of redundant data, thereby decreasing a speed of data acquisition and transmission, speed of image reconstruction, increasing data storage pressure, etc. Thus, it is desired to provide systems and methods for data acquisition and transmission with an improved efficiency.
US 2008/317194 A1 discloses a method for reconstructing list mode data acquired during a positron emission tomography scan of an object, the data including information indicative of a plurality of detected positron annihilation events. The method comprises: identifying detected list mode events occurring in a region of interest; reconstructing the identified list mode events using an iterative reconstruction technique which includes a ray tracing operation to generate tomographic data indicative of the region of interest, wherein the ray tracing operation traces only image matrix elements located in the region of interest; and generating an image indicative of the tomographic data. US 2012/089015 A1 discloses a method for reconstructing list mode data, the method comprising: reconstructing all list mode data of a list mode data set to generate a first reconstructed image; selecting a sub-set of the list mode data set; and reconstructing the sub-set of the list mode data set to generate an enhanced reconstructed image. US 2010/202664 A1 discloses a method of compensating for a motion of an object, the method comprising: modeling, in a first projection of projection data indicative of radionuclide decays in the object, a motion of the object; using the modeled motion to apply a spatial correction to projection data of the first projection.

### SUMMARY

The invention is set out in the appended set of claims.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary positron emission computed tomography (PET) system according to some embodiments of the present disclosure;
FIG. 2A is schematic diagram illustrating the PET 110 scanner according to some embodiments of the present disclosure;
FIG. 2B is a cross section of the PET scanner 110 according to some embodiments of the present disclosure;
FIG. 3 is a schematic diagram illustrating exemplary hardware and/or software components of a computing device on which a processing device may be implemented according to some embodiments of the present disclosure;
FIG. 4 is a block diagram illustrating an exemplary processing device according to some embodiments of the present disclosure;
FIG. 5 is a flowchart illustrating an exemplary process for image data acquisition according to some embodiments of the present disclosure;
FIG. 6 is a radial cross-view of an exemplary detector ring 600 of a PET scanner according to some embodiments of the present disclosure;
FIG. 7 is an axial cross-view of an exemplary detector assembly 700 of a PET scanner according to some embodiments of the present disclosure;
FIG. 8A is a schematic diagram illustrating an exemplary coincidence detection apparatus 800 according to some embodiments of the present disclosure;
FIG. 8B is a schematic diagram illustrating an exemplary coincidence detection component according to some embodiments of the present disclosure;
FIG. 8C is a schematic diagram illustrating an exemplary coincidence detection component according to some embodiments of the present disclosure;
FIG. 8D is a schematic diagram illustrating an exemplary coincidence detection component according to some embodiments of the present disclosure;
FIG. 9 is a flowchart illustrating an exemplary process for obtaining target coincidence events corresponding to an ROI of an object according to some embodiments of the present disclosure;
FIG. 10 is a flowchart illustrating an exemplary process for image data acquisition according to some embodiments of the present disclosure;
FIG. 11 is a flowchart illustrating an exemplary process for image data transmission according to some embodiments of the present disclosure;
FIG. 12 is a schematic diagram illustrating an exemplary first data transmitting link and an exemplary second data transmitting link according to some embodiments of the present disclosure; and
FIG. 13 is a schematic diagram illustrating an exemplary third data transmitting link according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well known methods, procedures, systems, components, and/or circuitry have been described at a relatively high-level, without detail, in order to avoid unnecessarily obscuring aspects of the present disclosure. The present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

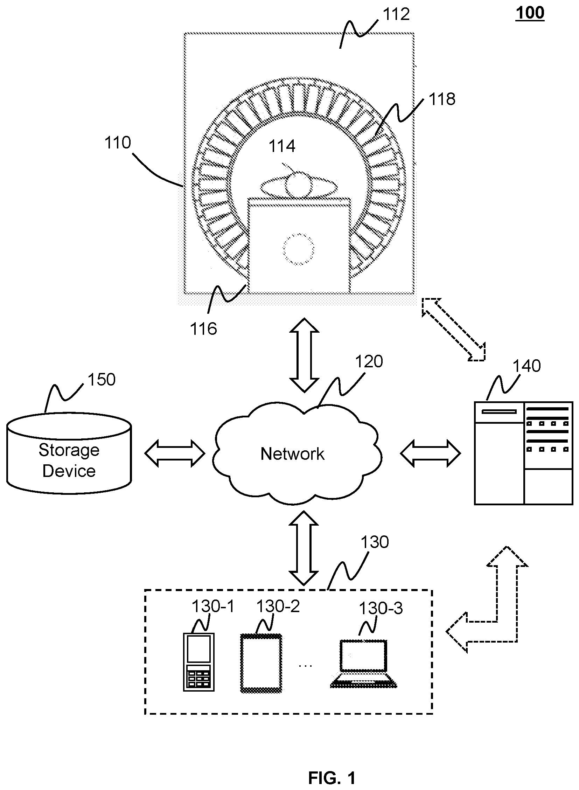

FIG. 1 is a schematic diagram illustrating an exemplary positron emission computed tomography (PET) system according to some embodiments of the present disclosure. PET imaging is based on coincidence events corresponding to detected photons arising from positron-electron annihilation. It should be noted that the PET system is merely provided for the purposes of illustration, and not intended to limit the scope of the present disclosure. For example, the PET system may be a multi-modality system. Exemplary multi-modality systems may include a magnetic resonance-positron emission tomography (MR-PET) system, a positron emission tomography-computed tomography (PET-CT) system, etc.

The PET system 100 may include a PET scanner 110, a network 120, a terminal 130, a processing device 140, and a storage device 150.

The PET scanner 110 may include a gantry 112, a table 116, a detector assembly 118, an electronics assembly, and other components (not shown). In some embodiments, when the PET scanner 110 performs a PET scan, an object 114 injected with a substance (e.g., generally necessary for the metabolism of biological life, such as glucose, protein, nucleic acids, fatty acids, etc.) that is labeled with a tracer for the imaging purposes may be placed on the table 116. The object 114 may be biological or non-biological. Merely by way of example, the object 114 may include a patient, a man-made object, etc. As another example, the object 114 may include a specific portion, organ, and/or tissue of the patient. For example, the object 114 may include the head, the brain, the neck, the body, a shoulder, an arm, the thorax, the heart, the stomach, a blood vessel, a soft tissue, a knee, a foot, or the like, of a patient, or any combination thereof.

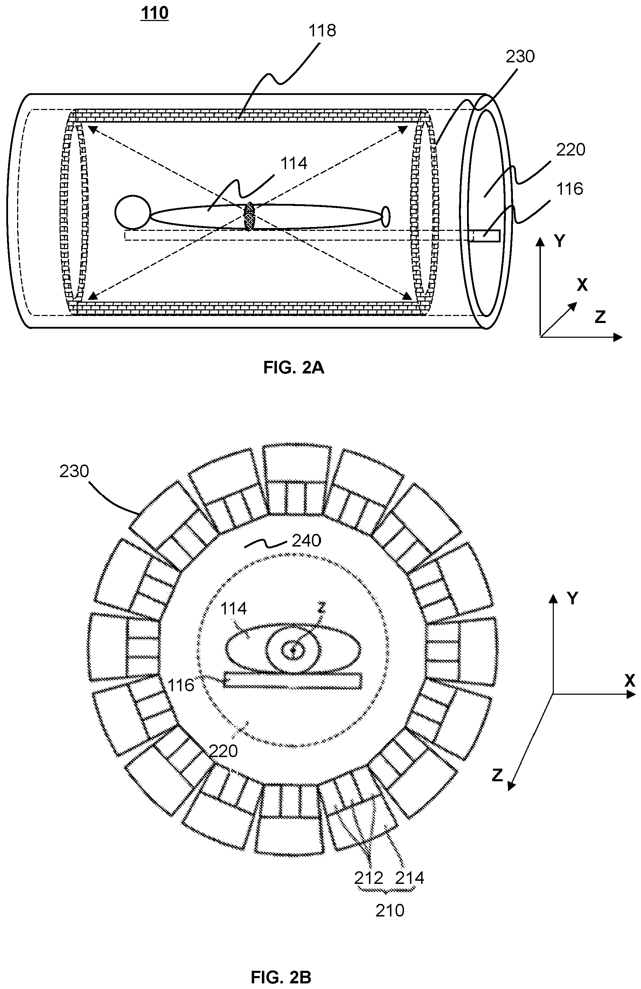

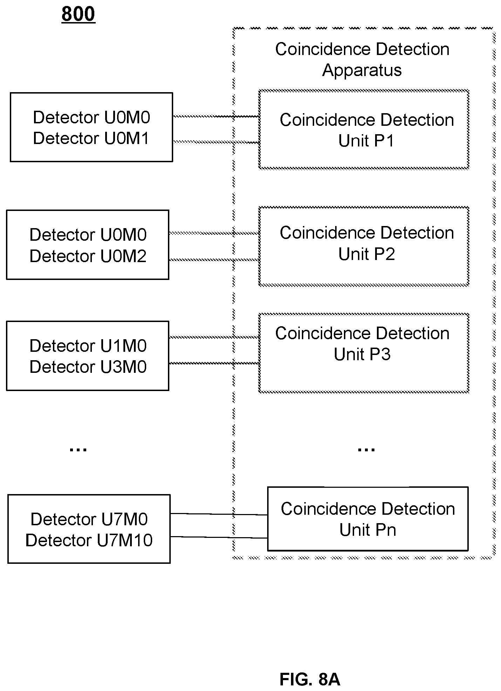

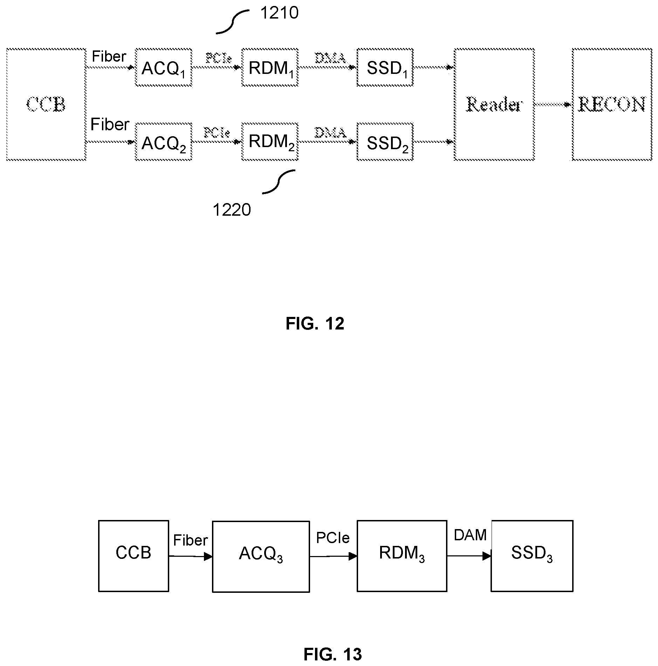

The gantry 112 may support one or more parts of the PET scanner 110, for example, the detector assembly 118, an electronics assembly, and/or other components. The detector assembly 118 may detect radiation photons (e.g., γ photons) emitted from an object being examined. The detector assembly 118 may include a plurality of detectors. The electronics assembly may include a coincidence detection apparatus and a data transmitting link. The coincidence detection apparatus may be configured to determine coincident events by processing outputs (e.g., electrical signals (e.g., pulses) of the detector assembly 118. The data transmitting link may be configured to transmit the coincidence events to a processing device that is same as or different from the processing device 140 for PET image reconstruction. As used herein, a radiation event (also referred to as a single event) may refer to a process that one of two radiation photons generated by an annihilation reaction in an object impinges a detector and is detected by the detector. A process that two radiation photons (e.g., γ photons) interacts with two detector blocks along a line of response (LOR) within a coincidence time window may be determined as a coincidence event (e.g., a truth coincidence event, a random coincidence event, a scatter coincidence event). More descriptions for the coincidence detection apparatus may be found elsewhere in the present disclosure (e.g., FIGs. 8A-8C). More descriptions for the data transmitting link may be found elsewhere in the present disclosure (e.g., FIGs. 12 and 13). More descriptions for the detector assembly 118 may be found elsewhere in the present disclosure (e.g., FIGs. 2A and 2B).

The network 120 may facilitate exchange of information and/or data. In some embodiments, one or more components in the PET system 100 (e.g., the PET scanner 110, the terminal 130, the processing device 140, or the storage device 150) may send information and/or data to other component(s) in the PET system 100 via the network 120. For example, the processing device 140 may obtain image data from the PET scanner 110 via the network 120. As another example, the processing device 140 may obtain user instructions from the terminal 130 via the network 120. In some embodiments, the network 120 may be any type of wired or wireless network, or combination thereof.

The terminal 130 may include a mobile device 130-1, a tablet computer 130-2, a laptop computer 130-3, or the like, or any combination thereof. In some embodiments, the terminal 130 may receive information and/or instructions inputted by a user, and transmit the received information and/or instructions to the PET scanner 110 or to the processing device 140 via the network 120. In some embodiments, the terminal 130 may receive data and/or information from the processing device 140. In some embodiments, the terminal 130 may be part of the processing device 140. In some embodiments, the terminal 130 may be omitted.

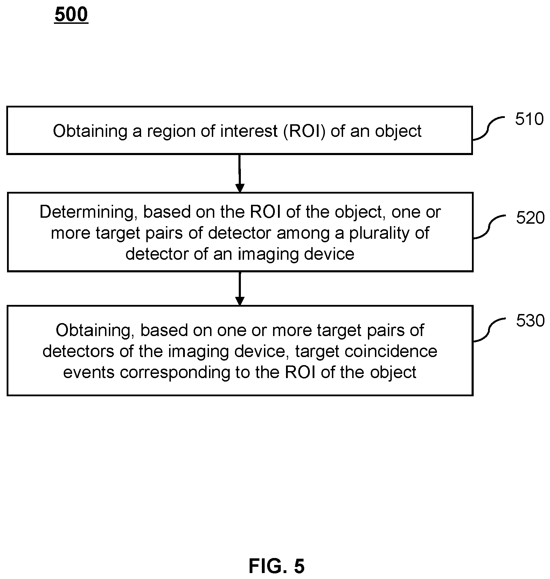

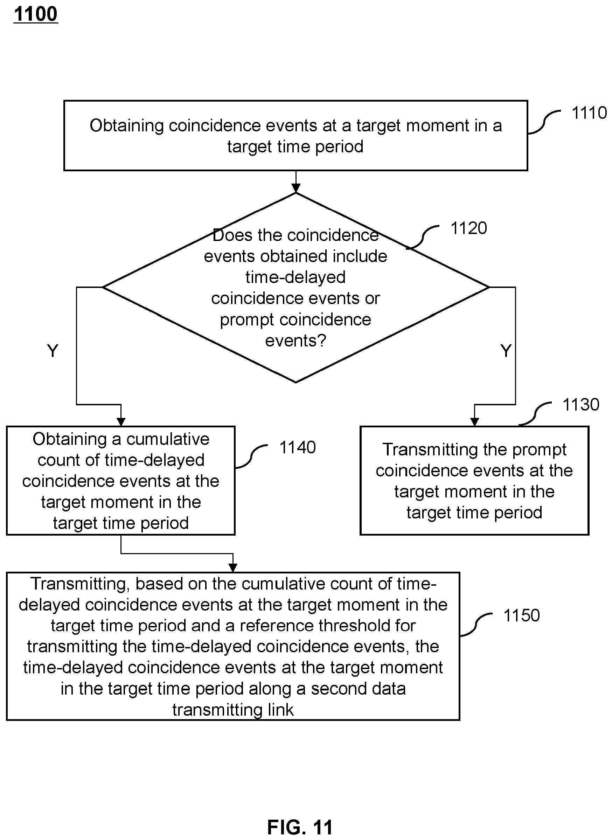

The processing device 140 may process data and/or information obtained from the PET scanner 110, the terminal 130, or the storage device 150. For example, the processing device 140 may cause the coincidence detection apparatus to obtain and/or output target coincidence events of a region of interest (ROI) of an object as described elsewhere in the present disclosure (e.g., FIGs. 5, 9 and 10). As another example, the processing device 140 may transmit coincidence events (e.g., target coincidence events) as described elsewhere in the present disclosure (e.g., FIGs. 11-13).

The storage device 150 may store data and/or instructions. In some embodiments, the storage device 150 may store data obtained from the terminal 130 and/or the processing device 140. In some embodiments, the storage device 150 may store data and/or instructions that the processing device 140 may execute or use to perform exemplary methods described in the present disclosure. In some embodiments, the storage device 150 may be part of the processing device 140.

FIG. 2A is schematic diagram illustrating the PET scanner 110 according to some embodiments of the present disclosure. FIG. 2B is a cross section of the PET scanner 110 according to some embodiments of the present disclosure. As shown in FIGs. 2A and 2B, the detector assembly 118 may include a plurality of detector rings arranged along a Z-axial direction (also referred to as a direction of a Z axis). A detector ring may also be referred to as a detector unit. A detector ring (e.g., a detector ring 230) may include a plurality of detectors (e.g., a detector 210) arranged along the circumference of the detector ring 230 in a plane perpendicular to the Z-axial direction. The plane perpendicular to the Z-axial direction may be defined by an X axis and an Y axis. A region encircled by the detector rings may be a detection region 240. The detection region 240 may accommodate the object 114 to be scanned.

In some embodiments, a detector may include a scintillator 212 and a photodetector 214. The photodetector 214 may be operably coupled to the scintillator 212. In some embodiments, the scintillator 212 may include an array of scintillation crystals. In some embodiments, positrons emitted from the radiation may travel in the object 114 until encountering electrons. When a positron and an electron meet, an annihilation reaction may occur. The electron-positron annihilation reaction may simultaneously generate two photons (e.g., 511-kiloelectron volt (keV) gamma photons) traveling in opposite directions along a line (i.e., a line of response). The two photons may be detected by two detectors (also referred to as a pair of detectors). Each of the two photons generated by the electron-positron annihilation reaction may strike the scintillator 212 to produce a burst of a fluorescent light. The fluorescence light may transmit from the scintillator 212 to the photodetector 214. The fluorescence light may be converted to an electrical signal (e.g., an electrical pulse) by the photodetector 214. The electrical signal may be transmitted to other components of the PET scanner, such as a coincidence detection apparatus.

The detector assembly 118 may form a bore to accommodate the table 116. In some embodiments, the axial length of the detector assembly may relate to an axial field of view (AFOV) of the PET scanner. The AFOV may refer to a maximum length along the Z-axial direction of the detector assembly 118 to detect a coincidence event effectively. For instance, the AFOV of the detector assembly 118 may be in a range from 0.75 meters to 2 meters. In some embodiments, the AFOV of the detector assembly 118 may exceed 0.75 meters, or 1 meter, or 1.5 meters, or 2 meters. During a scan process, the object 114 along with the table 116 may be moved into the bore to position a region of interest (ROI) of the object 114 in the FOV 220.

In some embodiments, the plurality of detector rings may be numbered with first serial numbers along the Z-axial direction. For example, the plurality of detector rings may be numbered with U0, U1, U2, ..., UQ in sequence along the Z-axial direction. A first serial number of a detector ring may indicate a position of the detector ring on the Z-axial direction. Detectors in the same detector ring may have the same first serial number. In some embodiments, detectors in each of the plurality of detector rings may be numbered with second serial numbers along the circumferential direction of the detector ring. For example, the detectors may be numbered with M0, M1, M2, ..., MN in sequence along the circumferential direction of a detector ring. A second serial number of a detector in a detector ring may indicate a position of the detector in the detector ring on the circumferential direction (i.e., a position on the X-Y plane). A position of a detector in a space may be defined by a first serial number that indicate a Z-axial position and a second serial number that indicates an X-axial and Y axial position.

In the present disclosure, the X axis, the Y axis, and the Z axis shown in FIG. 2 may form an orthogonal coordinate system. The Z axis may be substantially parallel to a long axis of the table when the table enters the detection region 240. It should be noted that the orthogonal coordinate system as shown in FIGs. 2A and 2B is merely provided for the purposes of illustration, and not intended to limit the scope of the present disclosure.

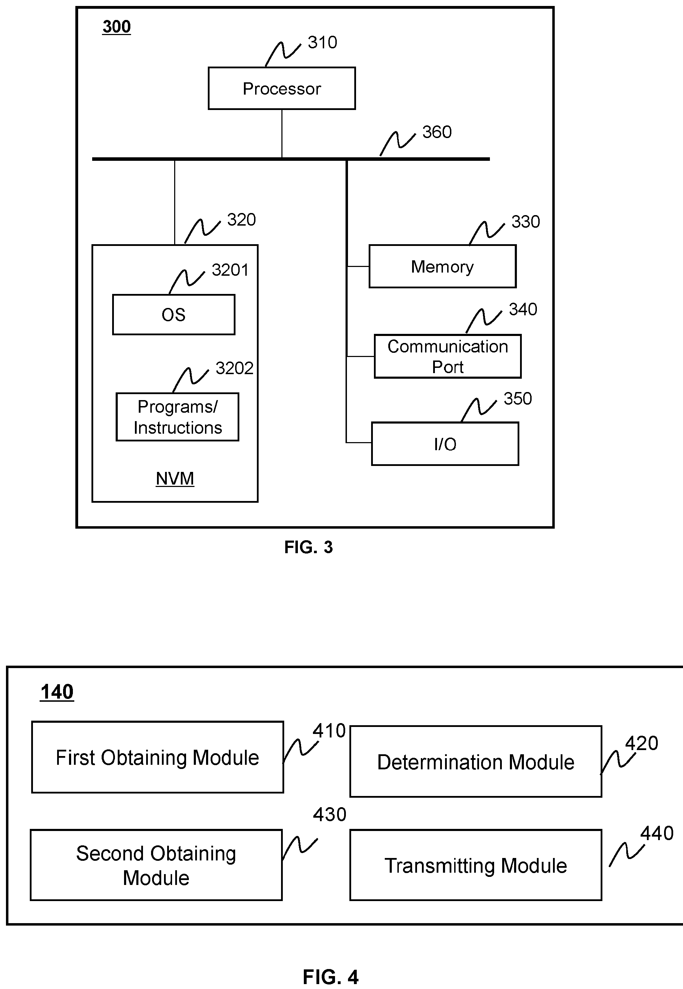

FIG. 3 is a schematic diagram illustrating exemplary hardware and/or software components of a computing device on which the processing device 140 may be implemented according to some embodiments of the present disclosure. As illustrated in FIG. 3, the computing device 300 may include a processor 310, nonvolatile memory (NVM) 320, memory 330, a communication port 340, and an input/output (I/O) 350 connected via a bus 360.

The processor 310 may execute computer instructions (program code) and perform functions of the processing device 140 in accordance with the present disclosure described herein. For example, the processor 310 may control a transmission of coincidence events obtained by the coincidence detection apparatus. As another example, the processor 320 may cause the coincidence detection apparatus to obtain target coincidence events corresponding to an ROI of the object.

The NVM 320 may store an operating system (OS) 3201 and one or more programs and/or instructions 3202 to be executed by the processor 310. The methods and/or processes of the present disclosure may be implemented as the program and/or instructions. For example, the NVM 320 may store a program for the processing device 140 for obtaining target coincidence events corresponding to an ROI of an object.

The memory 330 may support operations of the OS 3201 and the one or more programs and/or instructions 3202.

The communication port 340 may be connected to a network (e.g., the network 120) to facilitate data communications. The communication port 340 may establish connections between components in the processing device 140 and the PET scanner 110, the terminal 130, or the storage device 150.

The I/O 350 may input or output signals, data, or information. In some embodiments, the I/O 350 may enable a user interaction with the processing device 140 (i.e., the computing device 300). For example, the processing device 140 may display an image through the I/O 350. In some embodiments, the I/O 350 may include an input device and an output device. Exemplary input devices may include a keyboard, a mouse, a touch screen, a microphone, or the like, or a combination thereof. Exemplary output devices may include a display device, a loudspeaker, a printer, a projector, or the like, or a combination thereof.

FIG. 4 is a block diagram illustrating an exemplary processing device according to some embodiments of the present disclosure. The processing device 140 may include a first obtaining module 410, a determination module 420, a second obtaining module 430, and a transmitting module 440.

The first obtaining module 410 is configured to obtain a region of interest (ROI) of an object. An ROI of the object refers to a region of the object that a user (e.g., a doctor, a technician, an operator, etc.) focuses on. More descriptions regarding the obtaining of the ROI of the object may be found elsewhere in the present disclosure. See, e.g., operation 510 in FIG. 5, and relevant descriptions thereof.

The determination module 420 is configured to determine one or more target pairs of detectors among a plurality of detectors of an imaging device based on the ROI of the object. A response of line (LOR) corresponding to each pair of the one or more target pairs of detectors passes though the ROI when the object is located in a detection region of the imaging device. More descriptions regarding the determining of the one or more target pairs of detectors may be found elsewhere in the present disclosure. See, e.g., operation 520 in FIG. 5, and relevant descriptions thereof.

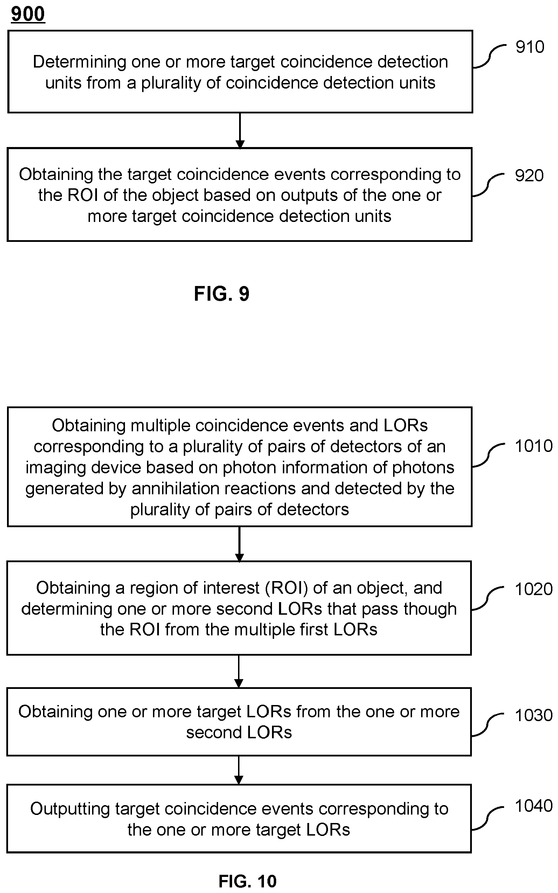

The second obtaining module may be configured to obtain target coincidence events corresponding to the ROI of the object based on the one or more target pairs of detectors of the imaging device. In some embodiments, the imaging device includes a coincidence detection apparatus. The coincidence detection apparatus is configured to determine coincidence events by processing outputs of the plurality of detectors. In some embodiments, the detection apparatus may include a plurality of coincidence detection components each of which is able to communicate with one pair of multiple pairs of detectors among the plurality of detectors via a wired connection or a wireless connection. In some embodiments, the processing device 140 determines one or more target coincidence detection components among the plurality of coincidence detection components. The one or more target coincidence detection components are in communication with the one or more target pairs of detectors. Further, the target coincidence events corresponding to the ROI of the object are obtained based on outputs of the one or more target coincidence detection components. More descriptions for the obtaining of the target coincidence events may be found elsewhere in the present disclosure. See, e.g., operation 520 in FIG. 5 and FIG. 9, and relevant descriptions thereof.

The transmitting module 440 may be configured to transmit coincidence events (e.g., the target coincidence events) to a terminal (e.g., the terminal 130) for storage or reconstructing an image (e.g., an image of the ROI) along a data transmitting link. More descriptions for the transmitting of the coincidence events may be found elsewhere in the present disclosure. See, e.g., FIG. 11 and relevant descriptions thereof.

FIG. 5 is a flowchart illustrating an exemplary process for image data acquisition according to some embodiments of the present disclosure. The process 500 may be implemented in the PET system 100 illustrated in FIG. 1. For example, the process 500 may be stored in the storage device150 and/or the storage 320 in the form of instructions (e.g., an application), and invoked and/or executed by the processing device 140 (e.g., the processor 310 illustrated in FIG. 3, or one or more modules in the processing device 140 illustrated in FIG. 4). The operations of the illustrated process presented below are intended to be illustrative. In some embodiments, the process 500 may be accomplished with one or more additional operations not described, and/or without one or more of the operations discussed. Additionally, the order in which the operations of the process 500 as illustrated in FIG. 5 and described below is not intended to be limiting.

In 510, the processing device 140 (e.g., the first obtaining module 410) obtains a region of interest (ROI) of an object.

An ROI of the object refers to a region of the object that a user (e.g., a doctor, a technician, an operator, etc.) focuses on. In some embodiments, the ROI of the object may be the entire object or a portion of the object. For example, the ROI of the object may include one or more specific organs and/or one or more specific tissues of, or the whole body of the object. Merely by way of example, the ROI may include the head, the chest, a lung, or the like, or any combination thereof, of a patient. In some embodiments, the ROI may include a lesion of the object. A lesion refers to a damage (or potential damage) and/or an abnormal change (or potential change) in the tissue of the object, usually caused by disease or trauma.

In some embodiments, the obtaining of an ROI may include determining a position of the ROI in a detection region of an imaging device. The imaging device may be configured to acquire imaging data relating to the object. In some embodiments, the imaging device may include a PET device (e.g., the PET scanner 110 described in FIG. 1). In some embodiments, the processing device 140 may determine the position of the ROI by determining a boundary range of the ROI in a coordinate system of the imaging device (e.g., the orthogonal coordinate system described in the FIGs. 2A and 2B). In some embodiments, the processing device 140 may determine the position of the ROI by determining a bounding box (e.g., a cube box) that encloses the boundary range of the ROI in the coordinate system of the imaging device (e.g., the orthogonal coordinate system described in the FIGs. 2A and 2B). The processing device 140 may designate the position of the bounding box as the position of the ROI.

In some embodiments, the processing device 140 may obtain a reference image including the ROI acquired by a reference imaging device. The processing device 140 may further determine the ROI of the object based on the reference image. The reference imaging device may be the same as or different from the imaging device. The determination of the ROI based on the reference image may include identifying the ROI from the reference image and determining the position of the ROI in the detection region of the imaging device based on the ROI represented in the reference image. For example, the processing device 140 may identify the ROI from the reference image and determine a position of the ROI in a coordinate system of the reference imaging device or the reference image. The processing device 140 may determine the position of the ROI in the detection region of the imaging device based on a coordinate transform relationship between the coordinate system of the imaging device and the coordinate system of the reference imaging device or the reference image.

In some embodiments, the reference image may include a pre-scanning image, a scout image, a diagnostic image, etc. For example, the pre-scanning image may be acquired by an MR device via scanning the object according to a fast pre-scanning pulse sequence. In some embodiments, the scout image may be acquired by the imaging device via scanning the object according to a positioning scanning technique. Exemplary positioning scanning techniques may include using camera imaging, infrared imaging, radiation imaging, etc. For example, the scout image may be acquired by an optical imaging device (e.g., a camera).

In some embodiments, the ROI may be identified from the reference image using an ROI identification technique (e.g., an image segmentation technique, a machine learning technique, etc.).

In some embodiments, the ROI of the object may be identified from the reference image manually by a user. For example, after the object is positioned in the detection region of the imaging device, the reference image may be displayed on a user interface. The user interface may be provided by the PET system 100 (e.g., the terminal 130) or an external device. In some embodiments, the reference image may represent a region corresponding to the detection region of the imaging device. A user may label the boundary of the ROI of the object on the reference image through an input device (e.g., a mouse, a keyboard, etc.) to determine the boundary range of the ROI. Alternatively, the user may label the ROI of the object (e.g., a region 610 or a region 710) on the reference image by moving a selection frame (e.g., the bounding box) through an input device.

In some embodiments, the processing device 140 may determine the position of the ROI in the detection region of the imaging device based on a mapping relationship (i.e., a coordinate transform relationship) between a position of a point in the reference image and a position of the point in the space wherein the imaging device is located. For each reference point representing the ROI in the reference image selected by the user, the processing device 140 may determine a position of the reference point in the detection region of the imaging device (i.e., in the coordinate system of the imaging device) based on the mapping relationship and a position of the reference point in the reference image. For example, the processing device 140 may obtain coordinates P1 of a reference point 1 in the reference image selected by the user. The processing device 140 may determine coordinates P1' based on the mapping relationship and the coordinates P1 of the reference point 1, and designate the coordinate P1' as coordinates of a point or a portion of the ROI in the space (or the detection region).

In 520, the processing device 140 (e.g., the determination module 420) determines, based on the ROI of the object, one or more target pairs of detectors among a plurality of detectors of an imaging device.

A line of response (LOR) corresponding to each pair of the one or more target pairs of detectors passes through the ROI (e.g., the boundary of the ROI or the bounding box enclosing the ROI) when the object is located in a detection region (i.e., the detection region) of the imaging device. As used herein, a line of response (LOR) corresponding to a pair of detectors refers to a line connecting the pair of detectors. An LOR corresponding to a pair of detectors may also be referred to as the LOR of the pair of detectors.

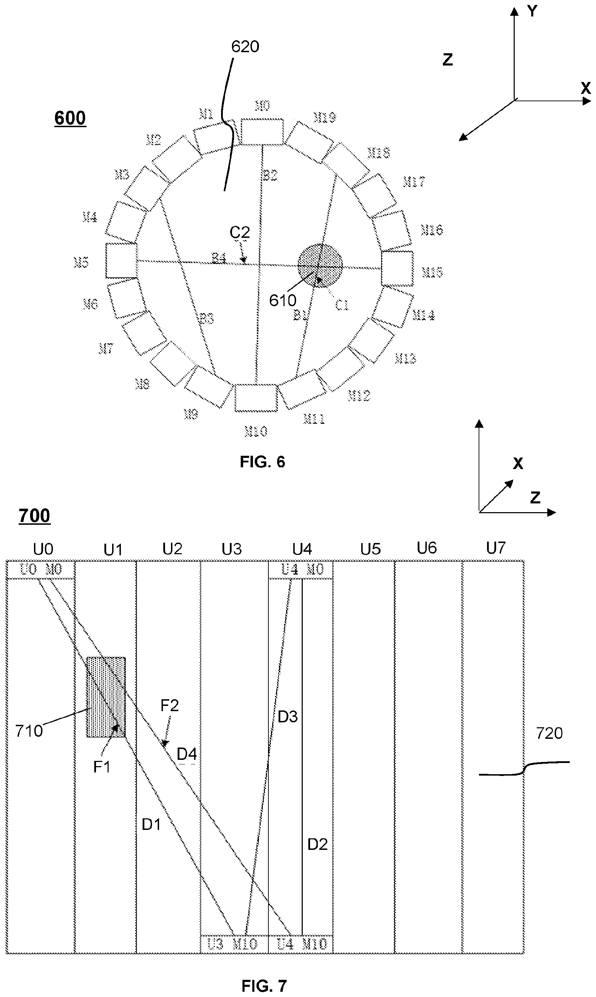

For example, FIG. 6 is a radial cross-view of an exemplary detector ring 600 of a PET scanner according to some embodiments of the present disclosure. As shown in FIG. 6, the detector ring 600 includes detectors M0-M19. An ROI 610 of an object is located within a detection region 620 of the detector ring 600. The processing device 140 may determine multiple LORs (e.g., lines B1, B2, B3, and B4) corresponding to any two of the detectors M0-M19 based on location information of the detectors M0-M19. For each of multiple LORs, the processing device 140 may determine whether an LOR traverses the ROI 610 of the object. In response to determine that the LOR traverses the ROI 610 of the object, the processing device 140 may determine a pair of detectors corresponding to the LOR as a target pair of detectors. For example, the lines B1 and B4 traverse the ROI 610 of the object, and the lines B2 and B2 do not traverse the ROI 610 of the object. The processing device 140 may determine the pair of detectors M11 and M18 corresponding to the lines B1 and the pair of detectors M5 and M15 corresponding to the lines B4 as target pairs of detectors.

As another example, FIG. 7 is an axial cross-view of an exemplary detector assembly 700 of a PET scanner according to some embodiments of the present disclosure. As shown in FIG. 7, the detector assembly 700 includes detector rings U0-U7. Each of the detector rings U0-U7 includes multiple detectors (e.g., a detector M0 of the detector ring U0). An ROI 710 of an object is located within a detection region 720 of the detector assembly 700. The processing device 140 may determine multiple target pairs of detectors in a similar manner described in FIG. 6. For example, the lines D1 and D4 traverse the ROI 710 of the object, and the lines D2 and D2 do not traverse the ROI 710 of the object. The processing device 140 may determine the pair of detectors U0M0 and U3M10 corresponding to the lines D1 and the pair of detectors U0M0 and U4M10 corresponding to the lines D4 as target pairs of detectors.

In some embodiments, the processing device 140 may determine the target LORs by simulation. For example, the processing device 140 may establish a three-dimensional (3D) model including the plurality of detectors in the imaging device and the ROI located within the detection region of the imaging device based on the position of the ROI. The processing device 140 may determine the target LORs by connecting each pair of detectors using a line in the 3D model and determining whether the line traverses the ROI (e.g., traversing the boundary of the ROI or the bounding box enclosing the ROI). In some embodiments, each LOR may have a geometric relationship for denoting positions of points on the LOR (e.g., a linear equation) in the coordinate system of the imaging apparatus, and the boundary of the ROI may have a geometric relationship for denoting positions of points on the boundary (e.g., a circular equation) in the coordinate system of the imaging apparatus. The processing device 140 may determine the target LORs based on the geometric relationships corresponding to the ROI and the LORs.

In 530, the processing device 140 (e.g., the second obtaining module 430) may obtain, based on one or more target pairs of detectors of the imaging device, target coincidence events corresponding to the ROI of the object.

The imaging device includes a coincidence detection apparatus. The coincidence detection apparatus is configured to determine coincidence events by processing outputs of the plurality of detectors. A process that two photons (also referred to as a pair of photons) detected by a pair of detectors within a time window may be considered to as a coincidence event.

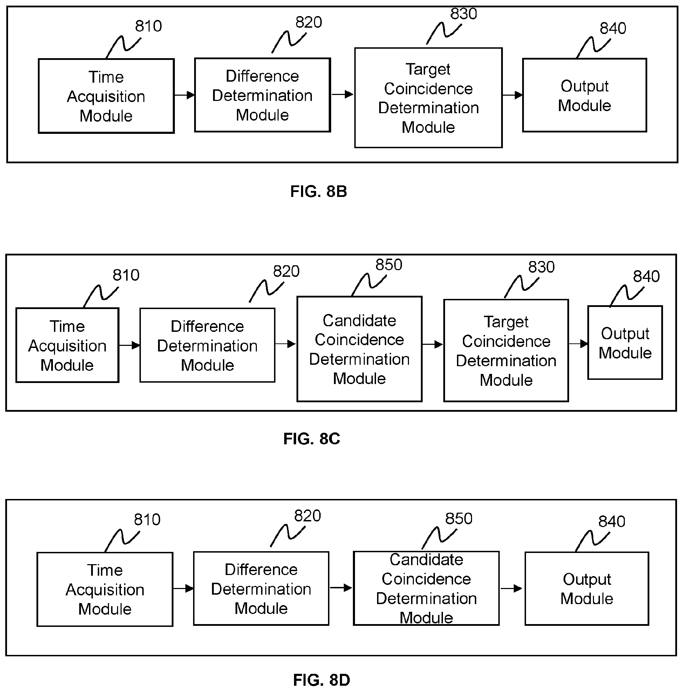

The detection apparatus includes a plurality of coincidence detection components (also referred to as coincidence detection sub-apparatuses) each of which is able to communicate with one pair of multiple pairs of detectors among the plurality of detectors via a wired connection or a wireless connection. For example, FIG. 8A is a schematic diagram illustrating an exemplary coincidence detection apparatus 800 according to some embodiments of the present disclosure. As shown in FIG. 8A, the detection apparatus 800 may include a plurality of coincidence detection components P1-Pn. Each of the plurality of coincidence detection components P1-Pn may be in communication with a pair of detectors. For example, the coincidence detection component P1 may be in communication with the pair of detectors U0M0 and U0M1, the coincidence detection component P2 may be in communication with the pair of detectors U0M0 and U0M2, etc. More descriptions for a coincidence detection component may be found elsewhere in the present disclosure. See, e.g., FIGs. 8A and 8D and relevant descriptions thereof.

According to the invention, the processing device 140 determines one or more target coincidence detection components among the plurality of coincidence detection components. The one or more target coincidence detection components are in communication with the one or more target pairs of detectors. Further, the target coincidence events corresponding to the ROI of the object are obtained based on outputs of the one or more target coincidence detection components. According to the invention, the outputs of the one or more target coincidence detection components are obtained by enabling the one or more target coincidence detection components to process outputs of the one or more target pairs of detectors when the imaging device scans the object; and preventing remaining coincidence detection components among the plurality of coincidence detection components excepting the one or more target coincidence detection components to process outputs of one or more remaining pairs of detectors excepting the one or more target pairs of detectors when the imaging device scans the object. More descriptions for the obtaining of the target coincidence events may be found elsewhere in the present disclosure. See, e.g., FIG. 9 and relevant descriptions thereof.

In some embodiments, after the target coincidence events are obtained, the processing device 140 may cause the coincidence detection apparatus (e.g., the one or more target coincidence detection components) to transmit the target coincidence events to a terminal (e.g., the terminal 130) for storage or reconstructing an image of the ROI along a data transmitting link.

In some embodiments, after the outputs (e.g., candidate coincidence events) of the one or more target coincidence units are obtained, the processing device 140 may determine the one or more target coincidence events from the outputs and transmit the target coincidence events to a terminal (e.g., the terminal 130) for storage or reconstructing an image of the ROI along a data transmitting link.

More descriptions for the transmitting of the target coincidence events may be found elsewhere in the present disclosure. See, e.g., FIGs. 11 and 12 and relevant descriptions thereof.

FIG. 9 is a flowchart illustrating an exemplary process for obtaining target coincidence events corresponding to an ROI of an object according to some embodiments of the present disclosure. In some embodiments, one or more operations of the process 900 may be performed to achieve at least part of operation 530 as described in connection with FIG. 5.

In 910, the processing device 140 (e.g., the second obtaining module 430) determines one or more target coincidence detection components from a plurality of coincidence detection components. Each of the one or more target coincidence detection components may be in communication with one of one or more target pairs of detectors via a wired connection or a wireless connection. An LOR corresponding to each of the one or more target pair of detectors may traverse an ROI of an object when a PET scanner scans the object located within a detection region of the PET scanner.

In some embodiments, a coincidence detection component may be in communication with a pair of detectors and configured to output coincidence events based on outputs (e.g., electrical signals) of the pair of detectors. The processing device 140 may determine one or more coincidence detection components in communication with the one or more target pairs of detectors as the one or more target coincidence detection components. More descriptions for the determining of the one or more target pairs of detectors may be found elsewhere in the present disclosure. See, e.g., operation 520 in FIG. 5 and relevant descriptions thereof.

In some embodiments, after the one or more target coincidence detection components are determined, the processing device 140 may control the operation of the plurality of coincidence detection components. The processing device 140 prevents the remaining coincidence detection components among the plurality of coincidence detection components excepting the one or more target coincidence detection components to output coincident events. According to the invention, the processing device 140 does not enable remaining coincidence detection components to process outputs of one or more remaining pairs of detectors excepting the one or more target pairs of detectors when the imaging device scans the object. As a further example, the processing device 140 may generate a first control signal and transmit the first control signal to each of the remaining coincidence detection components. The first control signal may stop the remaining coincidence detection components to work. The processing device 140 may enable the one or more target coincidence detection components to output coincident events. For example, the processing device 140 may enable the one or more target coincidence detection components to process outputs of the one or more target pairs of detectors when the imaging device scans the object. As a further example, the processing device 140 may generate a second control signal and transmit the second control signal to each of the target coincidence detection components. The second control signal may enable the target coincidence detection components to work.

In 920, the processing device 140 (e.g., the second obtaining module 430) obtains the target coincidence events corresponding to the ROI of the object based on outputs of the one or more target coincidence detection components.

In some embodiments, a target coincidence event may be a true coincident event, a random coincident event, a scattering coincident event, etc. A true coincident event may occur when two photons (a pair of photons) are generated by a same annihilation reaction, emitted from a same annihilation position (also referred to as a true annihilation position), and detected by a pair of detectors along an LOR within a certain coincidence time window (also referred to as a coincidence window range). A random coincident event may occur when two photons are generated by different annihilation reactions, emitted from different or the same annihilation position, and detected by a pair of detectors along an LOR within the certain coincidence time window. A scattering coincident event occurs when at least one of two photons detected by a pair of detectors along an LOR within the certain coincidence time window has undergone a Compton scattering prior to its detection. It should be noted that an annihilation position may also be determined based on information of the pair of photons in the random coincidence event, the scatter coincidence event, or radiation events detected by a pair of detectors, but the determined annihilation position is not real (also referred to as an equivalent annihilation position of an equivalent annihilation reaction). The equivalent annihilation position of an equivalent annihilation reaction of the pair of photons may also be referred to as the annihilation position of an annihilation reaction for generating the pair of photons.

In some embodiments, the outputs of the one or more target coincidence detection components may be the target coincidence events.

### Embodiment 1

In some embodiments, if a range of the ROI is smaller than a coincidence window range (or a coincidence window range encloses the range of ROI), an output of a target coincidence detection component may be generated by operations including: for a pair of multiple pairs of photons detected by a target pair of detectors communicating with the target coincidence detection component, obtaining, photon information of the pair of photons detected respectively by the target pair of detectors, determining whether an annihilation position (e.g., an equivalent annihilation position or a true annihilation position) corresponding to the pair of photons is located within the ROI based on the photon information of photons; and in response to determining that the annihilation position corresponding to the pair of photons is located within the ROI, outputting a coincidence event. As used herein, the coincidence window range may also be referred to as a first coincidence window that defines a coincidence event range (distance range). The coincidence event range may include be defined by a circle with a diameter that is equal to a multiplication between a lightspeed and a time coincidence window (also referred to as a second coincidence window). As used herein, the coincidence window range being greater than or equal to the range of ROI refers to that the boundary of the ROI is located within the coincidence event range defined by the first coincidence window. Accordingly, each pair of photons corresponding to an annihilation position that is determined based on photon information and located in the ROI may satisfy the coincidence window range and considered to correspond to a coincidence event.

For example, FIG. 8B is a schematic diagram illustrating an exemplary coincidence detection component according to some embodiments of the present disclosure. As shown in FIG. 8B, the coincidence detection component may include a time acquisition module 810, a difference determination module 820, a target coincidence determination module 830, and an output module 840. The time acquisition module 810 may be configured to obtain the photon information of the pair of photons (e.g., time information) that are detected by the target pair of detectors. The difference determination module 820 may be configured to determine a time difference between times when the pair of photons are detected by the target pair of detectors, respectively. The target coincidence determination module 830 may be configured to determine a target coincidence event by determining whether an annihilation position (e.g., an equivalent annihilation position or a true annihilation position) corresponding to the pair of photons is located within the ROI based on the photon information of photons (i.e., the time difference). If the annihilation position (e.g., an equivalent annihilation position or a true annihilation position) corresponding to the pair of photons is located within the ROI, the target coincidence determination module 830 may determine a target coincidence event corresponding to the pair of photons. The output module 840 may be configured to output the target coincidence event, for example, to a storage device or a processing device for image reconstruction along a data transmitting link.

In some embodiments, the target coincidence determination module 830 may determine the annihilation position based on a time difference between times when photons are detected by the target pair of detectors (i.e., the difference between detection times of the photons). For example, the photons detected by the target pair of detectors includes a first photon that is detected by a first detector among the target pair of detectors and a second photon that is detected by a second detector among the target pair of detectors. The time acquisition module 810 may obtain a first detection time of the first photon and a second detection time of the second photon, and the first detection time may be earlier than the second detection time. The difference determination module 820 may determine the difference *ΔT* between the first detection time and the second detection time. The target coincidence determination module 830 may determine a distance *L* between the target pair of detectors (i.e., a length of the LOR connecting the target pair of detectors). Photons may travel nearly at the lightspeed. Therefore, the target coincidence determination module 830 may determine that the annihilation position corresponding to the first photon and the second photon is a position having a distance *ΔL*/2 from the midpoint of the LOR, wherein, *ΔL*=*ΔT**lightspeed and the annihilation position may be near the first detector than the second detector. In response to determining that the annihilation position corresponding to the pair of photons is located within the ROI, the target coincidence determination module 830 may determine a target coincidence event corresponding to the pair of photons. The output module 840 may output the target coincidence event to for example, a storage device or a processing device for image reconstruction along a data transmitting link.

### Embodiment 2

In some embodiments, an output of a target coincidence detection component may be generated by operations including: determining candidate coincidence events by processing outputs of a target pair of detectors communicating with the target coincidence detection component; determining at least a portion of the candidate coincidence events as the output of the target coincidence detection component, wherein an annihilation position corresponding to each of the at least a portion of the candidate coincidence events is within the ROI; and outputting the at least a portion of the candidate coincidence events.

For example, FIG. 8C is a schematic diagram illustrating an exemplary coincidence detection component according to some embodiments of the present disclosure. As shown in FIG. 8C, the coincidence detection component may include a time acquisition module 810, a difference determination module 820, a target coincidence determination module 830, a candidate coincidence determination module 850 (also referred to as a coincidence determination module 850), and an output module 840.

The candidate coincidence determination module 850 may determine the candidate coincidence events. In some embodiments, a candidate coincidence event may be generated according to one or more of the following operations. The time acquisition module 810 may obtain photon information of photons that are emitted from the object and detected by a target pair of detectors communicating with a target coincidence detection component. The difference determination module 820 may obtain a coincidence window range (also referred to as a second coincidence window). The second coincidence window refers to a time range. The second coincidence window may be set manually by a user (e.g., an engineer) according to an experience value or a default setting of the PET system 100, or determined by the processing device 140 according to an actual need, such as 1 nanosecond, 2 nanoseconds, 5 nanoseconds, 10 nanoseconds, 20 nanoseconds, etc. Further, the candidate coincidence determination module 850 may determine a coincidence event based on the photon information of photons (i.e., a pair of photons) and the second coincidence window. For example, the candidate coincidence determination module 850 may determine whether the pair of photons corresponding to a coincidence event based on a time difference between detection times of the pair of photons detected by the target pair of detectors. A detection time of a photon refers to a time when the photon is detected by a detector. As a further example, the pair of photons detected by the target pair of detectors includes a first photon and a second photon. The time acquisition module 810 may determine a first detection time of the first photon and a second detection time of the second photon based on the photon information of photons. The difference determination module 820 may determine the time difference between the first detection time and the second detection time. The candidate coincidence determination module 850 may determine whether the time difference between the first detection time and the second detection time is within the second coincidence window. In response to determining that the time difference between the first detection time and the second detection time is within the second coincidence window, the candidate coincidence determination module 850 may determine that the first photon and the second photon satisfy a coincidence event (i.e., a candidate coincidence event), and output the coincidence event to the target coincidence determination module 830.

The target coincidence determination module 830 may determine at least a portion of the candidate coincidence events as the output (i.e., target coincide events) of the target coincidence detection component. In some embodiments, the target coincidence determination module 830 may determine a target coincidence event from the candidate coincidence events by determining whether an annihilation position of annihilation reaction corresponding to each of the candidate coincidence events is located within the ROI. In some embodiments, the target coincidence determination module 830 may determine an annihilation position of a candidate coincidence event based on the time difference between times when photons generated by the annihilation reaction are detected by the target pair of detectors (i.e., the difference between detection times of the photons). More descriptions for the determining of the annihilation position of annihilation reaction may be found elsewhere in the present disclosure. See, e.g., Example 1 and relevant descriptions thereof.

In some embodiments, in response to determining that the annihilation position corresponding to one of the candidate coincidence events is located within the ROI, the target coincidence determination module 830 may consider the one of the candidate coincidence events as a target coincidence event. For example, as shown in FIG. 6, an annihilation position C1 of annihilation reaction corresponding to a coincidence event on the LOR B1 is located within the ROI 610, and an annihilation position C2 of annihilation reaction corresponding to a coincidence event on the LOR B4 is not located within the ROI 610, the candidate coincidence determination module 850 may output the coincidence event on the LOR B1, and not output the coincidence event on the LOR B4. As another example, as shown in FIG. 7, an annihilation position F1 of annihilation reaction corresponding to a coincidence event on the LOR D1 is located within the ROI 710, and an annihilation position F2 of annihilation reaction corresponding to a coincidence event on the LOR D4 is not located within the ROI 710, the candidate coincidence determination module 850 may output the coincidence event on the LOR D1, and not output the coincidence event on the LOR D4.

### Embodiment 3

In some embodiments, the candidate coincidence events generated by the candidate coincidence determination module 850 may be outputted by the output module 840 as the target coincidence events.

For example, FIG. 8D is a schematic diagram illustrating an exemplary coincidence detection component according to some embodiments of the present disclosure. As shown in FIG. 8D, the coincidence detection component may include a time acquisition module 810, a difference determination module 820, a candidate coincidence determination module 850 (also referred to as a coincidence determination module 850), and an output module 840. Different from the coincidence detection component as shown in FIG. 8C, the coincidence detection component as shown in FIG. 8D does not include the target coincidence determination module 830.

Conventionally, a coincidence detection apparatus needs to process coincidence events generated by all detectors of an imaging device and output coincidence events corresponding to an object that locates in a detection region of the imaging region. Then, coincidence events corresponding to an ROI of the object can be obtained from the coincidence events corresponding to the object, which has a low efficiency. According to the present disclosure (e.g., the process 500 and process 900), the processing device 140 may determine one or more target pairs of detectors among a plurality of detectors of an imaging device based on the ROI of the object. Further, the processing device 140 may only enable the one or more target coincidence detection components to process outputs of generated by the one or more target pairs of detectors and prevent remaining coincidence detection components among the plurality of coincidence detection components excepting the one or more target coincidence detection components to process outputs of generated by one or more remaining pairs of detectors excepting the one or more target pairs of detectors when the imaging device scans the object. Compared with the conventional approach, according to some embodiments of the present disclosure, coincidence events output by the coincidence detection apparatus only includes target coincidence events corresponding to the ROI of the object and does not include coincidence events corresponding to other regions except the ROI, which may improve the efficiency of data acquisition, increase the speed of data acquisition, image reconstruction, and data storage, decrease data amount for data transmission, reduce the redundancy of data, relieve the pressure of data storage, and reduce the occupation of disk space.

It should be noted that the above description for the process 900 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, the processing device 140 may be integrated into the coincidence detection apparatus. For example, the processing device 140 may be integrated into each coincidence detection component in the coincidence detection apparatus. As a further example, the processing device 140 may include multiple control modules each of which is integrated into one of the plurality of coincidence detection components. A control module in a coincidence detection component may determine whether a pair of detectors communicating with the coincidence detection component is a target pair of detectors whose LOR traverses the ROI. If the pair of detectors communicating with the coincidence detection component is a target pair of detectors, the control module may enable other modules in the coincidence detection component to process outputs of the pair of detectors for determining coincidence events (e.g., a target coincidence event).

FIG. 10 is a flowchart illustrating an exemplary process for image data acquisition according to some embodiments of the present disclosure. The process 1000 may be implemented by a coincidence detection component.

In 1010, multiple coincidence events and LORs corresponding to a plurality of pairs of detectors of an imaging device may be obtained based on photon information of photons generated by annihilation reactions and detected by the plurality of pairs of detectors.

In some embodiments, the multiple coincidence events may be obtained by a coincidence detection component. More descriptions for the obtaining of the coincidence event and the LORs corresponding to the plurality of pairs of detectors may be found elsewhere in the present disclosure (e.g., FIG. 5 and FIG. 9 and the descriptions thereof).

In some embodiments, the processing device 140 may determine an LOR corresponding to each of the multiple coincidence events as a first LOR.

In 1020, a region of interest (ROI) of an object may be obtained, and one or more second LORs that pass though the ROI may be determined from the multiple first LORs.

In some embodiments, after the ROI of the object is determined by a user, the coincidence detection component may obtain the ROI of the object, and determine the one or more second LORs that pass though the ROI from the multiple first LORs.

In 1030, one or more target LORs may be obtained from the one or more second LORs. An annihilation position of an annihilation reaction corresponding to each of the one or more target LORs may be located within the ROI.

The coincidence detection component may determine whether an annihilation position of an annihilation reaction corresponding to each of the one or more second LORs is located within the ROI. More descriptions for the determining whether an annihilation position of an annihilation reaction is located within the ROI may be found elsewhere in the present disclosure (e.g., FIG. 9 and the descriptions thereof). In response to determining that an annihilation position of an annihilation reaction corresponding to a second LOR is located within the ROI, the second LOR may be determined as a target LOR.

In 1040, target coincidence events corresponding to the one or more target LORs may be output.

FIG. 11 is a flowchart illustrating an exemplary process for image data transmission according to some embodiments of the present disclosure. The process 1100 may be implemented in the PET system 100 illustrated in FIG. 1. For example, the process 1100 may be stored in the storage device 150 and/or the storage 320 in the form of instructions (e.g., an application), and invoked and/or executed by the processing device 140 (e.g., the processor 310 illustrated in FIG. 3, or one or more modules in the processing device 140 illustrated in FIG. 4).

In 1110, coincidence events may be obtained at a target moment (also referred to as a current moment) in a target time period (also referred to as a current time period). In some embodiments, the coincidence events may be obtained by a coincidence detection apparatus (e.g., implemented on the CCB as shown in FIGs. 12 and 13) as described elsewhere in the present disclosure. In some embodiments, the coincidence events may include target coincidence events corresponding to an ROI of an object that are obtained by target coincidence detection components in the coincidence detection apparatus as described elsewhere in the present disclosure (e.g., FIGs. 5 -10). The processing device 140 may cause the coincidence detection apparatus or the target coincidence detection components in the coincidence detection apparatus to obtain the coincidence events. More descriptions for the obtaining of the target coincidence events corresponding to the ROI of the object may be found elsewhere in the present disclosure (e.g., FIG. 5 and FIG. 9 and the descriptions thereof).

In some embodiments, the length of the target time period may be set manually by a user (e.g., an engineer) according to an experience value or a default setting of the PET system 100, or determined by the processing device 140 according to an actual need. For example, the length of the target time period may be 5s, 10s, etc. For example, the target time period may be from 10:01:01 to 10:01:15.

In some embodiments, the coincidence detection apparatus (e.g., the target coincidence detection components) may output the coincidence events (e.g., the target coincidence events) in real time. In some embodiments, the coincidence detection apparatus (e.g., the target coincidence detection components) may output the coincidence events (e.g., the target coincidence events) periodically. As used herein, "real time" may refer to a time interval between two adjacent time points for outputting data by the coincidence detection apparatus (e.g., a coincidence detection component) is less than a time threshold (e.g., 0.1s); "periodically" may refer to a time interval between two adjacent time points for outputting data by the coincidence detection apparatus (e.g., a coincidence detection component) exceeds a time threshold (e.g., 1s). For example, the coincidence detection apparatus (e.g., the target coincidence detection components) may output the coincidence events every 1 second. A target moment in the target time period refers to a time point in the target time period when one or more coincidence events are outputted by the coincidence detection apparatus (e.g., a target coincidence detection component).

In 1120, the processing device 140 (e.g., the transmitting module 440) may determining whether the coincidence events include time-delayed coincidence events or prompt coincidence events.

A coincident event may be a true coincident event, a random coincident event, a scattering coincident event, etc. A true coincident event may occur when two photons from a single annihilation reaction are detected by a pair of detectors along an LOR within a certain coincidence time window (also referred to as a coincidence window range). A random coincident event may occur when two photons from two separate annihilation reactions are detected by a pair of detectors along an LOR within the certain coincidence time window. A scattering coincident event occurs when at least one of two photons detected by a pair of detectors along an LOR within the certain coincidence time window has undergone a Compton scattering prior to its detection.

In some embodiments, a time-delayed coincidence event may refer to a random coincident event or a scattering coincident event, and a prompt coincidence event may refer to a true coincident event. In response to determining that the coincidence events outputted by the coincidence detection apparatus (e.g., a coincidence detection component) at the target moment are prompt coincidence events, the processing device 140 may perform operation 1130. In response to determining that the coincidence events outputted by the coincidence detection apparatus (e.g., a coincidence detection component) at the target moment are time-delayed coincidence events, the processing device 140 may perform operation 1140. In response to determining that the coincidence events outputted by the coincidence detection apparatus (e.g., a coincidence detection component) at the target moment include time-delayed coincidence events and prompt coincidence events, the processing device 140 may simultaneously perform operation 1130 and operation 1140.

In 1130, the processing device 140 (e.g., the transmitting module 440) may transmit the prompt coincidence events at the target moment in the target time period.

In some embodiments, the processing device 140 may transmit the prompt coincidence events in the coincidence events obtained in operation 1110 at the target moment in the target time period to a storage device or a processing device for image reconstruction (e.g., RECON as shown in FIG. 12) along a first data transmitting link. In some embodiments, the first data transmitting link may include one or more devices for transmitting the prompt coincidence events. For example, the first data transmitting link may include a first data acquisition hardware (e.g., a acquisition (ACQ) board), a first data acquisition software (e.g., a redis desktop manager (RDM)), and a first data storage device (e.g., a redis desktop manager (SSD)). The first data acquisition hardware may be configured to receive the prompt coincidence events output by the coincidence detection apparatus described in the FIG. 9 (e.g., a coincidence control board (CCB)) and transmit the prompt coincidence events to a computer device on which the first data acquisition software is installed. The data acquisition software may read the prompt coincidence events from the computer device and write the prompt coincidence events into the first data storage device (e.g., SSD). The first data storage device may be configured to store the prompt coincidence events written by the first data acquisition software.

In 1140, the processing device 140 (e.g., the transmitting module 440) may obtain a cumulative count of time-delayed coincidence events at the target moment in the target time period.

The cumulative count of time-delayed coincidence events at the target moment in the target time period refers to a count of time-delayed coincidence events from a starting moment of the target time period to the target moment. In some embodiments, the processing device 140 may determine whether the target moment in the target time period is a starting moment (i.e., a starting time point) in the target time period. In response to determining that the target moment in the target time period is the starting moment in the target time period, the processing device 140 may obtain an initial count of the cumulative count of time-delayed coincidence events in the target time period and obtain the cumulative count of time-delayed coincidence events at the target moment based on the initial count. In other words, the processing device 140 may update a cumulative count of time-delayed coincidence events in a previous time period of the target time period as the initial count. It should be noted that the updating the cumulative count of time-delayed coincidence events in the previous time period of the target time period as the initial count does not mean to remove or delete the cumulative count of time-delayed coincidence events in the previous time period, but the updating the cumulative count of time-delayed coincidence events in the previous time period is just used to obtain the initial count in the target time period.

In some embodiments, the processing device 140 may obtain an initial count of cumulative count of time-delayed coincidence events in the target time period according to actual needs. A cumulative count of time-delayed coincidence events at the starting moment in the target time period may be determined based on a count of time-delayed coincidence events obtained at the starting moment and the initial count of cumulative count of time-delayed coincidence events in the target time period. For example, the processing device 140 may designate the initial count of the cumulative count of time-delayed coincidence events in the target time period as 0. The processing device 140 may determine the count of time-delayed coincidence events obtained at the starting moment as the cumulative count of time-delayed coincidence events at the starting moment in the target time period. In some embodiments, the processing device 140 may designate the count of time-delayed coincidence events obtained at the target moment as the cumulative count of time-delayed coincidence events at the target moment in the target time period as the initial count of the cumulative count of time-delayed coincidence events in the target time period.

In some embodiments, in response to determining that the target moment in the target time period is not the starting moment in the target time period, the processing device 140 may obtain the cumulative count of time-delayed coincidence events at the target moment by updating the cumulative count of time-delayed coincidence events at an adjacent moment earlier than the target moment. Further, the cumulative count of time-delayed coincidence events at the target moment may be sum of the cumulative count of time-delayed coincidence events at the adjacent moment and the count of time-delayed coincidence events at the target moment. For example, if the target time period is from 10:01:01 to 10:01:15, the target moment is 10:01:09, an adjacent moment when time-delayed coincidence events (or coincidence events) are obtained earlier than the target moment may be 10:01:07. If the cumulative count of time-delayed coincidence events at 10:01:07 is 12 and a count of time-delayed coincidence events obtained at 10:01:09 is 3, the cumulative count of time-delayed coincidence events at 10:01:09 may be 15.

It should be understood that if the coincidence events obtained at a moment in the target time period do not include time-delayed coincidence events, the processing device 140 may do not update the cumulative count of time-delayed coincidence events at the moment, that is, the cumulative count of time-delayed coincidence events at the moment is the same as the cumulative count of time-delayed coincidence events at an adjacent moment when coincidence events are obtained earlier than the moment.

In 1150, the processing device 140 (e.g., the transmitting module 440) may transmit, based on the cumulative count of time-delayed coincidence events at the target moment in the target time period and a reference threshold for transmitting the time-delayed coincidence events, the time-delayed coincidence events at the target moment in the target time period along a second data transmitting link.

In some embodiments, the reference threshold may be related to a throughput of the second data transmitting link. The throughput of the second data transmitting link refers to a count of successfully transmitted data per unit of time for the second data transmitting link. In some embodiments, the reference threshold may be smaller than or equal to a product of the throughput of the second data transmitting link and the time length of the target time period.

In some embodiments, the second data transmitting link may be the same as or similar to the first data transmitting link. The second data transmitting link may include one or more devices for transmitting the time-delayed coincidence events. For example, the second data transmitting link may include a second data acquisition hardware (e.g., an ACQ board), a second data acquisition software (e.g., an RDM), and a second data storage device (e.g., an SSD). The second data acquisition hardware may be configured to receive the time-delayed coincidence events output by the coincidence detection apparatus described in the FIG. 9 (e.g., a CCB) and transmit the time-delayed coincidence events to a computer device on which the second data acquisition software is installed. The second data acquisition software may read the time-delayed coincidence events from the computer device and write the time-delayed coincidence events into the second data storage device (e.g., SSD). The second data storage device may be configured to store the time-delayed coincidence events written by the second data acquisition software.

In some embodiments, the processing device 140 may obtain a maximum processing speed of each of the one or more devices for transmitting the time-delayed coincidence events on the second data transmitting link. Exemplary one or more maximum processing speeds of the one or more devices for transmitting the time-delayed coincidence events may include a maximum transmission speed, a maximum reading speed, a maximum writing speed, a maximum storage speed, or the like, or any combination thereof. In some embodiments, the reference threshold may be determined based on at least one of the one or more maximum processing speeds of the one or more devices. In some embodiments, the reference threshold may be determined based on at least one of a maximum transmission speed, a maximum reading speed, a maximum writing speed, or a maximum storage speed of the second data transmitting link. For example, the second data transmitting link may include the second data acquisition hardware, the second data acquisition software, and the second data storage device. The maximum transmission speed (also referred to as a maximum transmission speed of the second data acquisition hardware) refers to a maximum speed at which the second data acquisition hardware transmits the time-delayed coincidence events to a computer device on which the second data acquisition software is installed. The maximum reading speed (also referred to as a maximum reading speed of the second data acquisition software) refers to a maximum speed at which the second data acquisition software reads the time-delayed coincidence events from the computer device. The maximum writing speed (also referred to as a maximum writing speed of the second data acquisition software) refers to a maximum speed at which the second data acquisition software writes the time-delayed coincidence events into the second data storage device. The maximum storage speed (also referred to as a maximum storage speed of the second data storage device) refers to a maximum speed at which the second data storage device store the time-delayed coincidence events.

For example, the processing device 140 may designate a product of the maximum writing speed and a time length of the target time period as the reference threshold. In this case, if one or more of the maximum transmission speed, the maximum reading speed, and the maximum storage speed is smaller than the maximum writing speed, for example, the maximum storage speed is smaller than the maximum writing speed, the data storage device on the second data transmitting link may be replaced to improve the maximum storage speed of the second data transmitting link.

In some embodiments, the reference threshold may be determined based on a minimum among the one or more maximum processing speeds of the one or more devices for transmitting the time-delayed coincidence events. The reference threshold may be smaller than or equal to a product of the time length of the target time period and the minimum among the one or more maximum processing speeds of the one or more devices for transmitting the time-delayed coincidence events. For example, the reference threshold may be determined based on a minimum among the maximum transmission speed, the maximum reading, the maximum writing speed, and the maximum storage speed. For example, the processing device 140 may determine a product of the time length of the target time period and the minimum among the maximum transmission speed, the maximum reading, the maximum writing speed, and the maximum storage speed as the reference threshold.

For illustration purposes, exemplary embodiments of the one or more maximum processing speeds of the one or more devices for transmitting the time-delayed coincidence events including a maximum transmission speed, a maximum reading speed, a maximum writing speed, a maximum storage speed are provided hereinafter.

In some embodiments, if the second data transmitting link and the first data transmitting link are two separate data transmitting links, the reference threshold may be determined based on at least one of the maximum transmission speed, the maximum reading speed, the maximum writing speed, or the maximum storage speed of the second data transmitting link.

For example, FIG. 12 is a schematic diagram illustrating an exemplary first data transmitting link and an exemplary second data transmitting link according to some embodiments of the present disclosure. As shown in FIG. 12, the first data transmitting link 1210 may include a ACQ₁ board, an RDM₁, and a SSD₁. The ACQ₁ board may receive the prompt coincidence events output by a CCB via an optical fiber cable and transmit the prompt coincidence events to a first computer device on which the RDM₁ is installed via a peripheral component interconnect express (PCIe). The RDM₁ may read the prompt coincidence events from the first computer device and write into the SSD₁ via a direct memory access (DMA). The second data transmitting link 1220 may include a ACQ₂ board, an RDM₂, and a SSD₂. The ACQ₂ board may receive the time-delayed coincidence events output by the CCB via an optical fiber cable and transmit the time-delayed coincidence events to a second computer device on which the RDM₂ is installed via a PCIe. The RDM₂ may read the time-delayed coincidence events from the second computer device and write into the SSD₂ via a DMA. After coincidence events are transmitted, a Data Reader may read the prompt coincidence events from the SSD₁ and time-delayed coincidence events from the SSD₂, and combine the prompt coincidence events and time-delayed coincidence events and transmit to a processing device *RECON* for image reconstruction. The reference threshold may be determined based on at least one of a maximum transmission speed of the ACQ₂ board, a maximum reading speed of the RDM₂, a maximum writing speed of the RDM₂, or a maximum storage speed of the SSD₂. For example, the processing device 140 may designate a product of the time length of the target time period and a minimum among the maximum transmission speed of the ACQ₂ board, the maximum reading speed of the RDM₂, the maximum writing speed of the RDM₂, and the maximum storage speed of the SSD₂ as the reference threshold.

In some embodiments, if the second data transmitting link and the first data transmitting link are a same data transmitting link, that is, prompt coincidence events and time-delayed coincidence events are transmitted along the same data transmitting link, the reference threshold may be determined based on a ratio of a count of the time-delayed coincidence events and a count of the prompt coincidence events in coincidence events and at least one of a maximum transmission speed, a maximum reading speed, a maximum writing speed, or a maximum storage speed of the same data transmitting link. The ratio of the count of the time-delayed coincidence events and the count of the prompt coincidence events may be determined by a user (e.g., an engineer) according to an experience value or a default setting of the PET system 100, or determined by the processing device 140 according to an actual need, such as 7:3, 6:4, etc.

For example, FIG. 13 is a schematic diagram illustrating an exemplary third data transmitting link according to some embodiments of the present disclosure. The prompt coincidence events and time-delayed coincidence events may be transmitted along the third data transmitting link. As shown in FIG. 3, the third data transmitting link may include a ACQ₃ board, an RDM₃, and a SSD₃. The ACQ₃ board may receive the prompt coincidence events and the time-delayed coincidence events output by the CCB via an optical fiber cable and transmit the prompt coincidence events and the time-delayed coincidence events to a third computer device on which the RDM₃ is installed via a PCIe. The RDM₃ may read the prompt coincidence events and the time-delayed coincidence events from the third computer device and write into the SSD₃ via a DMA. The reference threshold may be determined based on the ratio of the count of the time-delayed coincidence events and the count of the prompt coincidence events in the coincidence events and at least one of a maximum transmission speed of the ACQ₃ board, a maximum reading speed of the RDM₃, a maximum writing speed of the RDM₃, or a maximum storage speed of the SSD₃. For example, the maximum transmission speed of the ACQ₃ board is 15GB/s, the maximum reading speed of the RDM₃ is 20GB/s, the maximum writing speed of the RDM₃ is 10GB/s, the maximum storage speed of the SSD₃ is 30GB/s, and the ratio of the count of the time-delayed coincidence events and the count of the prompt coincidence events is 7:3. The processing device 140 may determine a product of the maximum writing speed of the RDM₃ and the ratio of the count of the time-delayed coincidence events and the count of the prompt coincidence events (i.e., 7 GB/s). The reference threshold may be smaller than or equal to a product of 7 GB/s and the time length of the target time period.

In some embodiments, if the second data transmitting link and the first data transmitting link are a same data transmitting link, the reference threshold may be determined based on a maximum transmission speed of the prompt coincidence events along the same data transmitting link and at least one of a maximum transmission speed, a maximum reading speed, a maximum writing speed, or a maximum storage speed of the same data transmitting link. The maximum transmission speed of the prompt coincidence events may be determined by a user (e.g., an engineer) according to an experience value or a default setting of the PET system 100, or determined by the processing device 140 according to an actual need.

In some embodiments, the processing device 140 may determine a minimum among the maximum transmission speed, the maximum reading, the maximum writing speed, and the maximum storage speed of the same data transmitting link. The processing device 140 may determine a difference between the minimum among the maximum transmission speed, the maximum reading, the maximum writing speed, and the maximum storage speed of the same data transmitting link and the maximum transmission speed of the prompt coincidence events along the same data transmitting link. The reference threshold may be smaller than or equal to a product of the time length of the target time period and the difference between the minimum among the maximum transmission speed, the maximum reading, the maximum writing speed, and the maximum storage speed of the same data transmitting link and the maximum transmission speed of the prompt coincidence events along the same data transmitting link.

For example, as shown in FIG. 13, the maximum transmission speed of the ACQ₃ board is 15GB/s, the maximum reading speed of the RDM₃ is 20GB/s, the maximum writing speed of the RDM₃ is 10GB/s, the maximum storage speed of the SSD₃ is 30GB/s, and the maximum transmission speed of the prompt coincidence events is 6 GB/s. The processing device 140 may determine that a maximum transmission speed of the time-delayed coincidence events is smaller than or equal to a difference between the maximum writing speed of the RDM₃ and the maximum transmission speed of the prompt coincidence events (i.e., 4 GB/s). The reference threshold may be smaller than or equal to a product of 4 GB/s and the time length of the target time period.

In some embodiments, the processing device 140 may determine whether the cumulative count of time-delayed coincidence events at the target moment in the target time period exceeds the reference threshold. In response to determining that the cumulative count of time-delayed coincidence events at the target moment in the target time period does not exceed the reference threshold, the processing device 140 may transmit the time-delayed coincidence events at the target moment in the target time period to a storage device or a processing device for image reconstruction along the second data transmitting link. In response to determining that the cumulative count of time-delayed coincidence events at the target moment in the target time period exceeds the reference threshold, the processing device 140 may stop transmitting time-delayed coincidence events obtained during a period after the target moment in the target time period, that is, the time-delayed coincidence events obtained during a period after the target moment in the target time period are not transmitted to the storage device or the processing device for image reconstruction along the second data transmitting link. But the processing device 140 may still obtain the cumulative count of time-delayed coincidence events during the period after the target moment in the target time period.

For example, the target time period is from 10:01:01 to 10:01:15, the reference threshold is 8, and the target moment is 10:01:12. If the cumulative count of time-delayed coincidence events at 10:01:12 is 7, the processing device 140 may determine that the cumulative count (i.e., 7) of time-delayed coincidence events at 10:01:12 does not exceed the reference threshold (i.e., 8), and transmit the time-delayed coincidence events at 10:01:12 to the storage device or the processing device for image reconstruction along the second data transmitting link. If the cumulative count of time-delayed coincidence events at 10:01:12 is 10, the processing device 140 may determining that the cumulative count (i.e., 10) of time-delayed coincidence events at 10:01:12 exceeds the reference threshold (i.e., 8), and stop transmitting time-delayed coincidence events obtained during a period from 10:01:12 to 10:01:15.

In some embodiments, the processing device 140 may determine whether a ratio of the cumulative count of time-delayed coincidence events at the target moment in the target time period to the reference threshold is greater than 1. In response to determining that the ratio of the cumulative count of time-delayed coincidence events at the target moment in the target time period to the reference threshold is not greater than 1, the processing device 140 may transmit the time-delayed coincidence events at the target moment in the target time period to a storage device or a processing device for image reconstruction along the second data transmitting link. In response to determining that the ratio of the cumulative count of time-delayed coincidence events at the target moment in the target time period to the reference threshold is greater than 1, the processing device 140 may stop transmitting time-delayed coincidence events obtained during a period after the target moment in the target time period.

In some embodiments, after the coincidence events in the target time period are transmitted, the processing device 140 may obtain and record the cumulative count of time-delayed coincidence events at an ending moment of the target time period in raw data. The raw data may include the transmitted prompt coincidence events, the transmitted time-delayed coincidence events, and the cumulative count of time-delayed coincidence events at the ending moment of the target time period.

In some embodiments, if the raw data only includes a portion of the time-delayed coincidence events in the coincidence events, the processing device 140 may compensate the raw data based on the cumulative count of time-delayed coincidence events in the raw data.

Merely by way of example, after the coincidence events in the target time period are transmitted, the processing device 140 may determine a ratio of the cumulative count of time-delayed coincidence events obtained in the target time period to a count of the transmitted time-delayed coincidence events corresponding to the target time period in the raw data. If the ratio of the cumulative count of time-delayed coincidence events obtained in the target time period to the count of the transmitted time-delayed coincidence events corresponding to the target time period in the raw data is 1:1, it indicates that all time-delayed coincidence events corresponding to the target time period are transmitted. If the ratio of the cumulative count of time-delayed coincidence events to the count of the transmitted time-delayed coincidence events corresponding to the target time period in the raw data is greater than 1:1, it indicates that only a portion of the time-delayed coincidence events corresponding to the target time period are transmitted. In this case, the processing device 140 may further compensate the raw data based on the ratio of the cumulative count of time-delayed coincidence events to the count of the transmitted time-delayed coincidence events corresponding to the target time period in the raw data.

For example, if the ratio of the cumulative count of time-delayed coincidence events to the count of the transmitted time-delayed coincidence events corresponding to the target time period in the raw data is 10:1, it indicates that only one-tenth of time-delayed coincidence events corresponding to the target time period are transmitted. The processing device 140 may multiply pixel values or voxel values corresponding to the time-delayed coincidence events corresponding to the target time period in the raw data by 10 to obtain compensated raw data corresponding to the target time period.

In some embodiments, after all coincidence events (e.g., the target coincidence events corresponding to the ROI of the object) are transmitted, the processing device 140 may reconstruct an image of the object (e.g., an image of the ROI of the object) based on the compensated raw data. In some embodiments, the processing device 140 may generate the image of the object by performing a reconstruction operation on the compensated raw data using an image reconstruction technique. Exemplary image reconstruction techniques may include an iterative reconstruction algorithm, a Fourier slice theorem algorithm, a filtered back projection (FBP) algorithm, or the like, or any combination thereof. It should be noted that the above reconstruction techniques are merely provided for the purposes of illustration, and not intended to limit the scope of the present disclosure.

Prompt coincidence events are vital to the accuracy of a reconstructed PET image of an object. Time-delayed coincidence events have an exponential relationship with a reaction activity of a substance labeled with a positron radioactive element injected into the object (i.e., a count of annihilation reactions in the object), and prompt coincidence events has a linear relationship with the reaction activity of the substance. That is, when the count of annihilation reactions is great (i.e., high activity acquisition), the count of the time-delayed coincidence events may be far greater than the count of prompt coincidence events. In this case, the accuracy of a PET image of an object reconstructed based on a portion of the time-delayed coincidence events and all prompt coincidence events can satisfy clinical requirements (e.g., requirements for disease diagnosis).

According to the present disclosure (e.g., the process 1100), if the cumulative count of time-delayed coincidence events at the target moment in the target time period does not exceed the reference threshold, the processing device 140 may transmit the obtained time-delayed coincidence events, and if the cumulative count of time-delayed coincidence events at the target moment in the target time period exceeds the reference threshold, the processing device 140 may stop transmitting time-delayed coincidence events obtained during a period after the target moment in the target time period, which may decrease data amount for data transmission, thereby improving the efficiency of data transmission, increasing the speed of image reconstruction and data storage, relieving the pressure of data storage, and reducing the occupation of disk space.

In addition, the transmission process of the coincidence events may include multiple time periods, the processing device 140 may transmit all prompt coincidence events and at least a portion of the time-delayed coincidence events corresponding to each time period, therefore, the distribution of the transmitted time-delayed coincidence events is not be changed, which may ensure the accuracy of the reconstructed image of the object. Moreover, in some embodiments, the processing device 140 may compensate the raw data based on the cumulative count of time-delayed coincidence events in the raw data, and reconstruct the image of the object based on compensated raw data corresponding to the object, which may further ensure the accuracy of the reconstructed image of the object.

It should be noted that the above description for the process 1100 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, if a coincidence detection apparatus for obtaining coincidence events includes multiple coincidence detection components, one or more of the multiple coincidence detection components may correspond to a data transmitting link for transmitting coincidence events, that is, the coincidence detection apparatus may correspond to one or more data transmitting link for transmitting coincidence events.
In some embodiments, the numbers expressing quantities, properties, and so forth, used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

## Claims

1. A system, comprising:
at least one storage device (150) including a set of instructions; and
at least one processor (310) in communication with the at least one storage device (150), wherein when executing the set of instructions, the at least one processor (310) is directed to perform operations including:
obtaining a region of interest, ROI, of an object;
determining, based on the ROI of the object, one or more target pairs of detectors among a plurality of detectors of an imaging device (110), wherein a line that connects each pair of the one or more target pairs of detectors passes through the ROI when the object is located in a detection region of the imaging device (110), the imaging device (110) includes a coincidence detection apparatus (800) configured to determine coincidence events by processing outputs of the plurality of detectors, the coincidence detection apparatus (800) includes a plurality of coincidence detection components each of which communicates with one pair of multiple pairs of detectors among the plurality of detectors;
determining one or more target coincidence detection components from the plurality of coincidence detection components, the one or more target coincidence detection components communicating with the one or more target pairs of detectors;
**characterized in that** the operations further include:
obtaining target coincidence events corresponding to the ROI of the object based on outputs of the one or more target coincidence detection components by:
enabling the one or more target coincidence detection components to process outputs of the one or more target pairs of detectors when the imaging device (110) scans the object; and
preventing remaining coincidence detection components among the plurality of coincidence detection components excepting the one or more target coincidence detection components to process outputs of one or more remaining pairs of detectors excepting the one or more target pairs of detectors when the imaging device (110) scans the object.

2. The system of claim 1, wherein
each of the outputs of the one or more target coincidence detection components includes multiple coincidence events on the line corresponding to one pair of the target pairs of detectors, and
the target coincidence events include coincidence events outputted by the one or more target coincidence detection components.

3. The system of claim 1, wherein the determining, based on the ROI of the object, one or more target pairs of detectors includes:
determining, based on location information of the plurality of detectors, multiple lines corresponding to multiple pairs of detectors among the plurality of detectors; and
determining one or more pairs of detectors whose lines pass though the ROI of the object as the one or more target pairs of detectors.

4. The system of any one of claims 1-3, wherein the at least one processor (310) is further directed to perform operations including:
determining whether the target coincidence events obtained at a target moment in a target time period include time-delayed coincidence events;
in response to determining that the target coincidence events obtained at the target moment in the target time period include time-delayed coincidence events, obtaining a cumulative count of time-delayed coincidence events at the target moment in the target time period; and
transmitting, based on the cumulative count of time-delayed coincidence events at the target moment in the target time period and a reference threshold for transmitting the time-delayed coincidence events, the time-delayed coincidence events at the target moment in the target time period along a data transmitting link.

5. The system of claim 4, wherein the at least one processor (150) is further directed to perform operations including:
determining whether the target coincidence events obtained at the target moment in the target time period include prompt coincidence events; and
in response to determining that the target coincidence events obtained at the target moment in the target time period include prompt coincidence events, transmitting the prompt coincidence events at the target moment in the target time period.

6. The system of claim 5, wherein the obtaining a cumulative count of time-delayed coincidence events at the target moment in the target time period includes:
determining whether the target moment in the target time period is a starting moment of the target time period; and
in response to determining that the target moment in the target time period is the starting moment of the target time period, obtaining an initial count of the cumulative count of time-delayed coincidence events at the starting moment in the target time period.

7. The system of claim 6, wherein the obtaining a cumulative value of time-delayed coincidence events at the target moment in the target time period includes:
in response to determining that the target moment in the target time period is not the starting moment of the target time period, obtaining the cumulative count of time-delayed coincidence events at the target moment by updating the cumulative count of time-delayed coincidence events at an adjacent moment earlier than the target moment.

8. The system of claim 5, wherein the transmitting, based on the cumulative count of time-delayed coincidence events at the target moment in the target time period and a reference threshold for transmitting the time-delayed coincidence events, the time-delayed coincidence events at the target moment in the target time period along a data transmitting link includes:
determining whether the cumulative count of time-delayed coincidence events at the target moment in the target time period exceeds the reference threshold; and
in response to determining that the cumulative count of time-delayed coincidence events at the target moment in the target time period does not exceed the reference threshold, transmitting the time-delayed coincidence events at the target moment in the target time period.

9. The system of claim 5, wherein the transmitting, based on the cumulative count of time-delayed coincidence events at the target moment in the target time period and a reference threshold for transmitting the time-delayed coincidence events, the time-delayed coincidence events at the target moment in the target time period along a data transmitting link includes:
determining whether the cumulative count of time-delayed coincidence events at the target moment in the target time period exceeds the reference threshold; and
in response to determining that the cumulative count of time-delayed coincidence events at the target moment in the target time period exceeds the reference threshold, stopping transmitting time-delayed coincidence events obtained during a period after the target moment in the target time period.

10. The system of claim 5, wherein the reference threshold is determined based on at least one of a maximum transmission speed, a maximum reading speed, a maximum writing speed, or a maximum storage speed of the data transmitting link.

11. A method, the method being implemented on a computing device (300) having at least one storage device (330) and at least one processor (310), the method comprising:
obtaining a region of interest (ROI) of an object;
determining, based on the ROI of the object, one or more target pairs of detectors among a plurality of detectors of an imaging device (110), wherein a line that connects each pair of the one or more target pairs of detectors passes though the ROI when the object is located in a detection region of the imaging device (110), the imaging device (110) includes a coincidence detection apparatus (800) configured to determine coincidence events by processing outputs of the plurality of detectors, the coincidence detection apparatus (800) includes a plurality of coincidence detection components each of which communicates with one pair of multiple pairs of detectors among the plurality of detectors;
determining one or more target coincidence detection components from the plurality of coincidence detection components, the one or more target coincidence detection components communicating with the one or more target pairs of detectors;
**characterized by** further comprising:
obtaining target coincidence events corresponding to the ROI of the object based on outputs of the one or more target coincidence detection components by:
enabling the one or more target coincidence detection components to process outputs of the one or more target pairs of detectors when the imaging device (110) scans the object; and
preventing remaining coincidence detection components among the plurality of coincidence detection components excepting the one or more target coincidence detection components to process outputs of one or more remaining pairs of detectors excepting the one or more target pairs of detectors when the imaging device (110) scans the object.

12. The method of claim 11, wherein
each of the outputs of the one or more target coincidence detection components includes multiple coincidence events on the line corresponding to one pair of the target pairs of detectors, and
the target coincidence events include coincidence events outputted by the one or more target coincidence detection components.

13. A non-transitory computer readable medium, comprising at least one set of instructions, wherein when executed by one or more processors (310) of a computing device (300), the at least one set of instructions causes the computing device (300) to perform a method, the method comprising:
obtaining a region of interest (ROI) of an object;
determining, based on the ROI of the object, one or more target pairs of detectors among a plurality of detectors of an imaging device (110), wherein a line that connects each pair of the one or more target pairs of detectors passes though the ROI when the object is located in a detection region of the imaging device (110), the imaging device (110) includes a coincidence detection apparatus (800) configured to determine coincidence events by processing outputs of the plurality of detectors, the coincidence detection apparatus (800) includes a plurality of coincidence detection components each of which communicates with one pair of multiple pairs of detectors among the plurality of detectors;
determining one or more target coincidence detection components from the plurality of coincidence detection components, the one or more target coincidence detection components communicating with the one or more target pairs of detectors;
**characterized in that** the method further comprises:
obtaining target coincidence events corresponding to the ROI of the object based on outputs of the one or more target coincidence detection components by:
enabling the one or more target coincidence detection components to process outputs of the one or more target pairs of detectors when the imaging device (110) scans the object; and
preventing remaining coincidence detection components among the plurality of coincidence detection components excepting the one or more target coincidence detection components to process outputs of one or more remaining pairs of detectors excepting the one or more target pairs of detectors when the imaging device (110) scans the object.

## Patentansprüche

1. System, umfassend:
mindestens eine Speichervorrichtung (150), die einen Satz von Anweisungen einschließt; und
mindestens einen Prozessor (310) in Kommunikation mit der mindestens einen Speichervorrichtung (150), wobei der mindestens einen Prozessor (310) beim Ausführen des Satzes von Anweisungen angewiesen ist, Operationen durchzuführen, die einschließen:
Bestimmen eines relevanten Bereichs, ROI, eines Objekts;
Bestimmen, auf der Grundlage des ROI des Objekts, eines oder mehrerer Zieldetektorpaare aus einer Vielzahl von Detektoren einer Bildgebungsvorrichtung (110), wobei eine Linie, die jedes Paar des einen oder der mehreren Zieldetektorpaare verbindet, durch den ROI verläuft, wenn sich das Objekt in einem Detektionsbereich der Bildgebungsvorrichtung (110) befindet, wobei die Bildgebungsvorrichtung (110) eine Koinzidenzdetektionseinrichtung (800) einschließt, die so konfiguriert ist, dass sie Koinzidenzereignisse durch Verarbeitung von Ausgaben der Vielzahl von Detektoren bestimmt, wobei die Koinzidenzdetektionseinrichtung (800) eine Vielzahl von Koinzidenzdetektionskomponenten einschließt, von denen jede mit einem Paar von mehreren Detektorpaaren aus der Vielzahl von Detektoren kommuniziert;
Bestimmen einer oder mehrerer Zielkoinzidenzdetektionskomponenten aus der Vielzahl der Koinzidenzdetektionskomponenten, wobei die eine oder mehreren Zielkoinzidenzdetektionskomponenten mit dem einen oder den mehreren Zieldetektorpaaren kommunizieren;
**dadurch gekennzeichnet, dass** die Operationen weiter Folgendes einschließen:
Erhalten von Zielkoinzidenzereignissen, die dem ROI des Objekts entsprechen, auf der Grundlage von Ausgaben der einen oder mehreren Zielkoinzidenzdetektionskomponenten durch:
Ermöglichen der Verarbeitung von Ausgaben des einen oder der mehreren Zieldetektorpaare durch die eine oder mehreren Zielkoinzidenzdetektionskomponenten, wenn die Bildgebungsvorrichtung (110) das Objekt abtastet; und
Verhindern, dass verbleibende Koinzidenzdetektionskomponenten aus der Vielzahl von Koinzidenzdetektionskomponenten, mit Ausnahme der einen oder mehreren Zielkoinzidenzdetektionskomponenten, Ausgaben eines oder mehrerer verbleibender Detektorpaare, mit Ausnahme des einen oder der mehreren Zieldetektorpaare, verarbeiten, wenn die Bildgebungsvorrichtung (110) das Objekt abtastet.

2. System nach Anspruch 1, wobei
jede der Ausgaben der einen oder mehreren Zielkoinzidenzdetektionskomponenten mehrere Koinzidenzereignisse auf der Linie einschließt, die einem Paar der Zieldetektorpaare entspricht, und
die Zielkoinzidenzereignissen Koinzidenzereignisse einschließen, die von der einen oder den mehreren Zielkoinzidenzdetektionskomponenten ausgegeben werden.

3. System nach Anspruch 1, wobei die Bestimmung eines oder mehrerer Zieldetektorpaare auf der Grundlage des ROI des Objekts Folgendes einschließt:
Bestimmen mehrerer Linien, die mehreren Detektorpaaren aus der Vielzahl den Detektoren entsprechen, auf der Grundlage von Standortinformationen der Vielzahl von Detektoren; und
Bestimmen eines oder mehrerer Detektorpaare, deren Linien durch den ROI des Objekts verlaufen, als das eine oder die mehreren Zieldetektorpaare.

4. System nach einem der Ansprüche 1-3, wobei der mindestens einen Prozessor (310) weiter angewiesen ist, Operationen durchzuführen, die Folgendes einschließen:
Bestimmen, ob die zu einem Zielzeitpunkt in einem Zielzeitraum erhaltenen Zielkoinzidenzereignisse zeitverzögerte Koinzidenzereignisse einschließen;
als Reaktion auf die Bestimmung, dass die zum Zielzeitpunkt im Zielzeitraum erhaltenen Zielkoinzidenzereignisse zeitverzögerte Koinzidenzereignisse einschließen, Erhalten einer kumulativen Anzahl von zeitverzögerten Koinzidenzereignisse zum Zielzeitpunkt im Zielzeitraum; und
Übertragen der zeitverzögerten Koinzidenzereignisse zum Zielzeitpunkt im Zielzeitraum über eine Datenübertragungsverbindung auf der Grundlage der kumulativen Anzahl von zeitverzögerten Koinzidenzereignisse zum Zielzeitpunkt im Zielzeitraum und einem Referenzschwellenwert für die Übertragung der zeitverzögerten Koinzidenzereignisse.

5. System nach Anspruch 4, wobei der mindestens einen Prozessor (150) weiter angewiesen ist, Operationen durchzuführen, die Folgendes einschließen:
Bestimmen, ob die zum Zielzeitpunkt im Zielzeitraum erhaltenen Zielkoinzidenzereignisse unmittelbare Koinzidenzereignisse einschließen; und
als Reaktion auf die Bestimmung, dass die zum Zielzeitpunkt im Zielzeitraum erhaltenen Zielkoinzidenzereignisse unmittelbare Koinzidenzereignisse einschließen, Übertragen der unmittelbaren Koinzidenzereignisse zum Zielzeitpunkt im Zielzeitraum.

6. System nach Anspruch 5, wobei das Erhalten einer kumulativen Anzahl zeitverzögerter Koinzidenzereignisse zum Zielzeitpunkt im Zielzeitraum Folgendes einschließt:
Bestimmen, ob der Zielzeitpunkt im Zielzeitraum ein Anfangszeitpunkt des Zielzeitraums ist; und
als Reaktion auf die Bestimmung, dass der Zielzeitpunkt im Zielzeitraum der Anfangszeitpunkt des Zielzeitraums ist, Erhalten einer anfänglichen Anzahl der kumulierten Anzahl zeitverzögerter Koinzidenzereignisse zum Anfangszeitpunkt im Zielzeitraum.

7. System nach Anspruch 6, wobei das Bestimmen eines kumulativen Wertes zeitverzögerter Koinzidenzereignisse zum Zielzeitpunkt im Zielzeitraum Folgendes einschließt:
als Reaktion auf die Bestimmung, dass der Zielzeitpunkt im Zielzeitraum nicht der Anfangszeitpunkt des Zielzeitraums ist, Erhalten der kumulativen Anzahl zeitverzögerter Koinzidenzereignisse zum Zielzeitpunkt durch Aktualisieren der kumulativen Anzahl zeitverzögerter Koinzidenzereignisse zu einem benachbarten Zeitpunkt vor dem Zielzeitpunkt.

8. System nach Anspruch 5, wobei die Übertragung der zeitverzögerten Koinzidenzereignisse zum Zielzeitpunkt im Zielzeitraum über eine Datenübertragungsverbindung auf der Grundlage der kumulativen Anzahl zeitverzögerter Koinzidenzereignisse zum Zielzeitpunkt im Zielzeitraum und eines Referenzschwellenwerts für die Übertragung der zeitverzögerten Koinzidenzereignisse Folgendes einschließt:
Bestimmen, ob die kumulative Anzahl zeitverzögerter Koinzidenzereignisse zum Zielzeitpunkt im Zielzeitraum den Referenzschwellenwert überschreitet; und
als Reaktion auf die Bestimmung, dass die kumulative Anzahl zeitverzögerter Koinzidenzereignisse zum Zielzeitpunkt im Zielzeitraum den Referenzschwellenwert nicht überschreitet, Übertragen der zeitverzögerten Koinzidenzereignisse zum Zielzeitpunkt im Zielzeitraum.

9. System nach Anspruch 5, wobei die Übertragung der zeitverzögerten Koinzidenzereignisse zum Zielzeitpunkt im Zielzeitraum über eine Datenübertragungsverbindung auf der Grundlage der kumulativen Anzahl zeitverzögerter Koinzidenzereignisse zum Zielzeitpunkt im Zielzeitraum und eines Referenzschwellenwerts für die Übertragung der zeitverzögerten Koinzidenzereignisse Folgendes einschließt:
Bestimmen, ob die kumulative Anzahl zeitverzögerter Koinzidenzereignisse zum Zielzeitpunkt im Zielzeitraum den Referenzschwellenwert überschreitet; und
als Reaktion auf die Bestimmung, dass die kumulative Anzahl zeitverzögerter Koinzidenzereignisse zum Zielzeitpunkt im Zielzeitraum den Referenzschwellenwert überschreitet, Beenden der Übertragung von zeitverzögerten Koinzidenzereignissen, die während eines Zeitraums nach dem Zielzeitpunkt im Zielzeitraum erhalten wurden.

10. System nach Anspruch 5, wobei der Referenzschwellenwert auf der Grundlage mindestens einer von einer maximalen Übertragungsgeschwindigkeit, einer maximalen Lesegeschwindigkeit, einer maximalen Schreibgeschwindigkeit oder einer maximalen Speichergeschwindigkeit der Datenübertragungsverbindung bestimmt wird.

11. Verfahren, wobei das Verfahren auf einer Rechenvorrichtung (300) implementiert ist, die mindestens eine Speichervorrichtung (330) und mindestens einen Prozessor (310) aufweist, wobei das Verfahren Folgendes umfasst:
Erhalten eines relevanten Bereichs (ROI) eines Objekts;
Bestimmen, auf der Grundlage des ROI des Objekts, eines oder mehreres Zieldetektorpaare aus einer Vielzahl von Detektoren einer Bildgebungsvorrichtung (110), wobei eine Linie, die jedes Paar des einen oder der mehreren Zieldetektorpaare verbindet, durch den ROI verläuft, wenn sich das Objekt in einem Detektionsbereich der Bildgebungsvorrichtung (110) befindet, wobei die Bildgebungsvorrichtung (110) eine Koinzidenzdetektionseinrichtung (800) einschließt, die so konfiguriert ist, dass sie Koinzidenzereignisse durch Verarbeiten von Ausgaben der Vielzahl von Detektoren bestimmt, wobei die Koinzidenzdetektionseinrichtung (800) eine Vielzahl von Koinzidenzdetektionskomponenten einschließt, von denen jede mit einem Paar von mehreren Detektorpaaren aus der Vielzahl von Detektoren kommuniziert;
Bestimmen einer oder mehrerer Zielkoinzidenzdetektionskomponenten aus der Vielzahl der Koinzidenzdetektionskomponenten, wobei die eine oder mehreren Zielkoinzidenzdetektionskomponenten mit dem einen oder den mehreren Zieldetektorpaaren kommunizieren;
**dadurch gekennzeichnet, dass** es weiter Folgendes umfasst:
Erhalten von Zielkoinzidenzereignissen, die dem ROI des Objekts entsprechen, auf der Grundlage von Ausgaben der einen oder mehreren Zielkoinzidenzdetektionskomponenten durch:
Ermöglichen der Verarbeitung von Ausgaben des einen oder der mehreren Zieldetektorpaare durch die eine oder mehreren Zielkoinzidenzdetektionskomponenten, wenn die Bildgebungsvorrichtung (110) das Objekt abtastet; und
Verhindern, dass verbleibende Koinzidenzdetektionskomponenten aus der Vielzahl von Koinzidenzdetektionskomponenten, mit Ausnahme der einen oder mehreren Zielkoinzidenzdetektionskomponenten, Ausgaben eines oder mehrerer verbleibender Detektorpaare, mit Ausnahme des einen oder der mehreren Zieldetektorpaare, verarbeiten, wenn die Bildgebungsvorrichtung (110) das Objekt abtastet.

12. Verfahren nach Anspruch 11, wobei
jede der Ausgaben der einen oder mehreren Zielkoinzidenzdetektionskomponenten mehrere Koinzidenzereignisse auf der Linie einschließt, die einem Paar der Zieldetektorpaare entspricht, und
die Zielkoinzidenzereignissen Koinzidenzereignisse einschließen, die von der einen oder den mehreren Zielkoinzidenzdetektionskomponenten ausgegeben werden.

13. Nichtflüchtiges computerlesbares Medium, umfassend mindestens einen Satz von Anweisungen, wobei der mindestens eine Satz von Anweisungen bei Ausführung durch einen oder mehrere Prozessoren (310) einer Rechenvorrichtung (300) bewirkt, dass die Rechenvorrichtung (300) ein Verfahren durchführt, wobei das Verfahren umfasst:
Erhalten eines relevanten Bereichs (ROI) eines Objekts;
Bestimmen, auf der Grundlage des ROI des Objekts, eines oder mehreres Zieldetektorpaare aus einer Vielzahl von Detektoren einer Bildgebungsvorrichtung (110), wobei eine Linie, die jedes Paar des einen oder der mehreren Zieldetektorpaare verbindet, durch den ROI verläuft, wenn sich das Objekt in einem Detektionsbereich der Bildgebungsvorrichtung (110) befindet, wobei die Bildgebungsvorrichtung (110) eine Koinzidenzdetektionseinrichtung (800) einschließt, die so konfiguriert ist, dass sie Koinzidenzereignisse durch Verarbeiten von Ausgaben der Vielzahl von Detektoren bestimmt, wobei die Koinzidenzdetektionseinrichtung (800) eine Vielzahl von Koinzidenzdetektionskomponenten einschließt, von denen jede mit einem Paar von mehreren Detektorpaaren aus der Vielzahl von Detektoren kommuniziert;
Bestimmen einer oder mehrerer Zielkoinzidenzdetektionskomponenten aus der Vielzahl der Koinzidenzdetektionskomponenten, wobei die eine oder mehreren Zielkoinzidenzdetektionskomponenten mit dem einen oder den mehreren Zieldetektorpaaren kommunizieren;
**dadurch gekennzeichnet, dass** das Verfahren weiter Folgendes umfasst:
Erhalten von Zielkoinzidenzereignissen, die dem ROI des Objekts entsprechen, auf der Grundlage von Ausgaben der einen oder mehreren Zielkoinzidenzdetektionskomponenten durch:
Ermöglichen der Verarbeitung von Ausgaben des einen oder der mehreren Zieldetektorpaare durch die eine oder mehreren Zielkoinzidenzdetektionskomponenten, wenn die Bildgebungsvorrichtung (110) das Objekt abtastet; und
Verhindern, dass verbleibende Koinzidenzdetektionskomponenten aus der Vielzahl von Koinzidenzdetektionskomponenten, mit Ausnahme der einen oder mehreren Zielkoinzidenzdetektionskomponenten, Ausgaben eines oder mehrerer verbleibender Detektorpaare, mit Ausnahme des einen oder der mehreren Zieldetektorpaare, verarbeiten, wenn die Bildgebungsvorrichtung (110) das Objekt abtastet.

## Revendications

1. Système, comprenant :
au moins un dispositif de stockage (150) incluant un ensemble d'instructions ; et
au moins un processeur (310) en communication avec le au moins un dispositif de stockage (150), dans lequel, lors de l'exécution de l'ensemble d'instructions, le au moins un processeur (310) est amené à effectuer des opérations incluant :
l'obtention d'une région d'intérêt, ROI, d'un objet ;
la détermination, sur la base de la ROI de l'objet, d'une ou plusieurs paires cibles de détecteurs parmi une pluralité de détecteurs d'un dispositif d'imagerie (110), dans lequel une ligne qui relie chaque paire des une ou plusieurs paires cibles de détecteurs passe par la ROI lorsque l'objet est situé dans une région de détection du dispositif d'imagerie (110), le dispositif d'imagerie (110) inclut un appareil de détection de coïncidence (800) configuré pour déterminer les événements de coïncidence en traitant les sorties de la pluralité de détecteurs, l'appareil de détection de coïncidence (800) inclut une pluralité de composants de détection de coïncidence, dont chacun communique avec une paire de multiples paires de détecteurs parmi la pluralité de détecteurs ;
la détermination d'un ou plusieurs composants de détection de coïncidence cibles parmi la pluralité de composants de détection de coïncidence, les un ou plusieurs composants de détection de coïncidence cibles communiquant avec les une ou plusieurs paires cibles de détecteurs ;
**caractérisé en ce que** les opérations incluent en outre :
l'obtention d'événements de coïncidence cibles correspondant à la ROI de l'objet sur la base de sorties des un ou plusieurs composants de détection de coïncidence cibles par :
l'autorisation des un ou plusieurs composants de détection de coïncidence cibles de traiter les sorties des une ou plusieurs paires cibles de détecteurs lorsque le dispositif d'imagerie (110) balaie l'objet ; et
l'empêchement des composants de détection de coïncidence restants parmi la pluralité de composants de détection de coïncidence, à l'exception des un ou plusieurs composants de détection de coïncidence cibles, de traiter les sorties d'une ou plusieurs paires restantes de détecteurs, à l'exception des une ou plusieurs paires cibles de détecteurs, lorsque le dispositif d'imagerie (110) balaie l'objet.

2. Système selon la revendication 1, dans lequel
chacune des sorties des un ou plusieurs composants de détection de coïncidence cibles inclut de multiples événements de coïncidence sur la ligne correspondant à une paire des paires cibles de détecteurs, et
les événements de coïncidence cibles incluent les événements de coïncidence générés par les un ou plusieurs composants de détection de coïncidence cibles.

3. Système selon la revendication 1, dans lequel la détermination, sur la base de la ROI de l'objet, d'une ou plusieurs paires cibles de détecteurs inclut :
la détermination, sur la base d'informations de localisation de la pluralité de détecteurs, de multiples lignes correspondant à de multiples paires de détecteurs parmi la pluralité de détecteurs ; et
la détermination d'une ou plusieurs paires de détecteurs dont les lignes traversent la ROI de l'objet comme étant les une ou plusieurs paires cibles de détecteurs.

4. Système selon l'une quelconque des revendications 1-3, dans lequel le au moins un processeur (310) est en outre amené à effectuer des opérations incluant
la détermination du fait que les événements de coïncidence cibles obtenus à un moment cible pendant une période de temps cible incluent ou non des événements de coïncidence retardés dans le temps ;
en réponse à la détermination que les événements de coïncidence cibles obtenus au moment cible pendant la période de temps cible incluent des événements de coïncidence retardés dans le temps, l'obtention d'un décompte cumulatif d'événements de coïncidence retardés dans le temps au moment cible pendant la période de temps cible ; et
la transmission, sur la base du décompte cumulatif d'événements de coïncidence retardés dans le temps au moment cible pendant la période de temps cible et d'un seuil de référence pour la transmission des événements de coïncidence retardés dans le temps, des événements de coïncidence retardés dans le temps au moment cible pendant la période de temps cible le long d'une liaison de transmission de données.

5. Système selon la revendication 4, dans lequel le au moins un processeur (150) est en outre amené à effectuer des opérations incluant :
la détermination du fait que les événements de coïncidence cibles obtenus au moment cible pendant la période de temps cible incluent ou non des événements de coïncidence rapides ; et
en réponse à la détermination que les événements de coïncidence cibles obtenus au moment cible pendant la période de temps cible incluent des événements de coïncidence rapides, la transmission des événements de coïncidence rapides au moment cible pendant la période de temps cible.

6. Système selon la revendication 5, dans lequel l'obtention d'un décompte cumulatif d'événements de coïncidence retardés dans le temps au moment cible pendant la période de temps cible inclut :
la détermination du fait que le moment cible pendant la période de temps cible est ou non un moment de début de la période de temps cible ; et
en réponse à la détermination du fait que le moment cible dans la période de temps cible est le moment de début de la période de temps cible, l'obtention d'un décompte initial du décompte cumulatif des événements de coïncidence retardés dans le temps au moment de début de la période de temps cible.

7. Système selon la revendication 6, dans lequel l'obtention d'une valeur cumulative d'événements de coïncidence retardés dans le temps au moment cible pendant la période de temps cible inclut :
en réponse à la détermination du fait que le moment cible pendant la période de temps cible n'est pas le moment de début de la période de temps cible, l'obtention du décompte cumulatif d'événements de coïncidence retardés dans le temps au moment cible en mettant à jour le décompte cumulatif d'événements de coïncidence retardés dans le temps à un moment adjacent antérieur au moment cible.

8. Système selon la revendication 5, dans lequel la transmission, sur la base du décompte cumulatif d'événements de coïncidence retardés dans le temps au moment cible pendant la période de temps cible et d'un seuil de référence pour la transmission des événements de coïncidence retardés dans le temps, des événements de coïncidence retardés dans le temps au moment cible pendant la période de temps cible le long d'une liaison de transmission de données inclut :
la détermination du fait que le décompte cumulatif d'événements de coïncidence retardés dans le temps au moment cible pendant la période de temps cible dépasse ou non le seuil de référence ; et
en réponse à la détermination du fait que le décompte cumulatif d'événements de coïncidence retardés dans le temps au moment cible pendant la période de temps cible ne dépasse pas le seuil de référence, la transmission des événements de coïncidence retardés dans le temps au moment cible pendant la période de temps cible.

9. Système selon la revendication 5, dans lequel la transmission, sur la base du décompte cumulatif d'événements de coïncidence retardés dans le temps au moment cible pendant la période de temps cible et d'un seuil de référence pour la transmission des événements de coïncidence retardés dans le temps, des événements de coïncidence retardés dans le temps au moment cible pendant la période de temps cible le long d'une liaison de transmission de données inclut :
la détermination du fait que le décompte cumulatif d'événements de coïncidence retardés dans le temps au moment cible pendant la période de temps cible dépasse ou non le seuil de référence ; et
en réponse à la détermination du fait que le décompte cumulatif d'événements de coïncidence retardés dans le temps au moment cible pendant la période de temps cible dépasse le seuil de référence, l'arrêt de la transmission des événements de coïncidence retardés dans le temps obtenus pendant une période après le moment cible pendant la période de temps cible.

10. Système selon la revendication 5, dans lequel le seuil de référence est déterminé sur la base d'au moins l'une parmi une vitesse de transmission maximale, une vitesse de lecture maximale, une vitesse d'écriture maximale ou une vitesse de stockage maximale de la liaison de transmission de données.

11. Procédé, le procédé étant mis en œuvre sur un dispositif informatique (300) présentant au moins un dispositif de stockage (330) et au moins un processeur (310), le procédé comprenant :
l'obtention d'une région d'intérêt (ROI) d'un objet ;
la détermination, sur la base de la ROI de l'objet, d'une ou plusieurs paires cibles de détecteurs parmi une pluralité de détecteurs d'un dispositif d'imagerie (110), dans lequel une ligne qui relie chaque paire des une ou plusieurs paires cibles de détecteurs passe par la ROI lorsque l'objet est situé dans une région de détection du dispositif d'imagerie (110), le dispositif d'imagerie (110) inclut un appareil de détection de coïncidence (800) configuré pour déterminer les événements de coïncidence en traitant les sorties de la pluralité de détecteurs, l'appareil de détection de coïncidence (800) inclut une pluralité de composants de détection de coïncidence, dont chacun communique avec une paire de multiples paires de détecteurs parmi la pluralité de détecteurs ;
la détermination d'un ou plusieurs composants de détection de coïncidence cibles parmi la pluralité de composants de détection de coïncidence, les un ou plusieurs composants de détection de coïncidence cibles communiquant avec les une ou plusieurs paires cibles de détecteurs ;
**caractérisé en ce qu'**il comprend en outre :
l'obtention d'événements de coïncidence cibles correspondant à la ROI de l'objet sur la base de sorties des un ou plusieurs composants de détection de coïncidence cibles par :
l'autorisation des un ou plusieurs composants de détection de coïncidence cibles de traiter les sorties des une ou plusieurs paires cibles de détecteurs lorsque le dispositif d'imagerie (110) balaie l'objet ; et
l'empêchement des composants de détection de coïncidence restants parmi la pluralité de composants de détection de coïncidence, à l'exception des un ou plusieurs composants de détection de coïncidence cibles, de traiter les sorties d'une ou plusieurs paires restantes de détecteurs, à l'exception des une ou plusieurs paires cibles de détecteurs, lorsque le dispositif d'imagerie (110) balaie l'objet.

12. Procédé selon la revendication 11, dans lequel
chacune des sorties des un ou plusieurs composants de détection de coïncidence cibles inclut de multiples événements de coïncidence sur la ligne correspondant à une paire des paires cibles de détecteurs, et
les événements de coïncidence cibles incluent les événements de coïncidence générés par les un ou plusieurs composants de détection de coïncidence cibles.

13. Support non transitoire lisible par ordinateur, comprenant au moins un ensemble d'instructions, dans lequel, lorsqu'il est exécuté par un ou plusieurs processeurs (310) d'un dispositif informatique (300), le au moins un ensemble d'instructions amène le dispositif informatique (300) à mettre en œuvre un procédé, le procédé comprenant :
l'obtention d'une région d'intérêt (ROI) d'un objet ;
la détermination, sur la base de la ROI de l'objet, d'une ou plusieurs paires cibles de détecteurs parmi une pluralité de détecteurs d'un dispositif d'imagerie (110), dans lequel une ligne qui relie chaque paire des une ou plusieurs paires cibles de détecteurs passe par la ROI lorsque l'objet est situé dans une région de détection du dispositif d'imagerie (110), le dispositif d'imagerie (110) inclut un appareil de détection de coïncidence (800) configuré pour déterminer les événements de coïncidence en traitant les sorties de la pluralité de détecteurs, l'appareil de détection de coïncidence (800) inclut une pluralité de composants de détection de coïncidence, dont chacun communique avec une paire de multiples paires de détecteurs parmi la pluralité de détecteurs ;
la détermination d'un ou plusieurs composants de détection de coïncidence cibles parmi la pluralité de composants de détection de coïncidence, les un ou plusieurs composants de détection de coïncidence cibles communiquant avec les une ou plusieurs paires cibles de détecteurs ;
**caractérisé en ce que** le procédé comprend en outre :
l'obtention d'événements de coïncidence cibles correspondant à la ROI de l'objet sur la base de sorties des un ou plusieurs composants de détection de coïncidence cibles par :
l'autorisation des un ou plusieurs composants de détection de coïncidence cibles de traiter les sorties des une ou plusieurs paires cibles de détecteurs lorsque le dispositif d'imagerie (110) balaie l'objet ; et
l'empêchement des composants de détection de coïncidence restants parmi la pluralité de composants de détection de coïncidence, à l'exception des un ou plusieurs composants de détection de coïncidence cibles, de traiter les sorties d'une ou plusieurs paires restantes de détecteurs, à l'exception des une ou plusieurs paires cibles de détecteurs, lorsque le dispositif d'imagerie (110) balaie l'objet.
